# FASCICULE DE BREVET EUROPEEN

(11) **EP 3 320 109 B1**
(45) Date de publication et mention de la délivrance du brevet: **18.03.2020**
(21) Numéro de dépôt: 16747827.0
(22) Date de dépôt: 06.07.2016
(51) Int. Cl.: C12Q 1/18, C12Q 1/04, C12Q 1/20

(54) **PROCEDE DE DETECTION D'UNE PRESENCE OU D'UNE ABSENCE D'AU MOINS UNE PREMIERE ZONE D'INHIBITION**
VERFAHREN ZUR ERKENNUNG DES VORHANDENSEINS ODER NICHTVORHANDENSEINS VON MINDESTENS EINER ERSTEN HEMMZONE
METHOD FOR DETECTING A PRESENCE OR ABSENCE OF AT LEAST A FIRST ZONE OF INHIBITION

(30) Priorité: 09.07.2015 FR 1556490
(43) Date de publication de la demande: 16.05.2018
(73) Titulaire: Biomérieux, 69280 Marcy l'Étoile (FR)
(72) Inventeur: DRAZEK, Laurent, 38100 Grenoble (FR); DUPONT-FILLARD, Agnès, 38190 Les Ardets (FR); PINSTON, Frédéric, 38000 Grenoble (FR); ROSTAING, Hervé, 38420 Le Versoud (FR)
(86) Numéro de dépôt international: PCT/FR2016/051712
(87) Numéro de publication internationale: WO 2017/006055

(56) Documents cités:
- WO-A1-00/55357
- WO-A1-99/45095
- WO-A2-2013/160408
- FR-A1- 2 988 400
- US-A- 5 028 529
- US-A1- 2010 099 137
- US-A1- 2013 029 371
- "Etest(R) Déterminer la Concentration Minimale Inhibitrice (CMI) d'un antibiotique, d'un antifongique ou d'un antituberculeux (BioMérieux)", , 1 juin 2015 (2015-06-01), XP055271602, Extrait de l'Internet: URL:http://www.biomerieux.fr/diagnostic-cl inique/etest [extrait le 2016-05-10]
- JAMES Q BOEDICKER ET AL: "Detecting bacteria and determining their susceptibility to antibiotics by stochastic confinement in nanoliter droplets using plug-based microfluidics", LAB ON A CHIP: MINIATURISATION FOR CHEMISTRY, PHYSICS, BIOLOGY, MATERIALS SCIENCE AND BIOENGINEERING, ROYAL SOCIETY OF CHEMISTRY, GB, vol. 8, no. 8, 4 juillet 2008 (2008-07-04) , - 1 août 2008 (2008-08-01), pages 1265-1272, XP007914504, ISSN: 1473-0197, DOI: 10.1039/B804911D
- LARYSA BARABAN ET AL: "Millifluidic droplet analyser for microbiology", LAB ON A CHIP, vol. 11, no. 23, 20 octobre 2011 (2011-10-20), pages 4057-4062, XP055080174, ISSN: 1473-0197, DOI: 10.1039/c1lc20545e

## Description

Le domaine technique de la présente invention est celui de la microbiologie. Plus particulièrement, la présente invention concerne la détection d'une présence ou d'une absence d'au moins une première zone d'inhibition d'un échantillon contenant ou susceptible de contenir des microorganismes en présence d'un agent chimique.

Plus particulièrement, la présente invention concerne les tests de détermination de la sensibilité d'un microorganisme à un antibiotique.

Un test classique de sensibilité est le test de diffusion en disque, souvent désigné comme la méthode de Kirby-Bauer. Cette méthode standardisée implique l'ensemencement d'un milieu de culture gélosé (par exemple une gélose Mueller-Hinton de 90mm ou 150mm) par un échantillon généralement standardisé à 0,5 Mc Farland, obtenu à partir d'un isolat microbien. L'ensemencement peut être réalisé par les méthodes manuelles classiques à l'aide d'un écouvillon ou d'une oese. Alternativement, l'ensemencement peut être réalisé par inondation de la gélose avec une suspension standardisée à un dixième de 0,5 Mc Farland, suivi du retrait de l'excédent d'échantillon. A la suite de l'ensemencement, un ou plusieurs disques de papier imprégnés avec des concentrations définies d'antibiotiques sont disposés à la surface de la gélose. Après une période d'incubation, généralement de 16 à 20 heures à 35°C, le diamètre de la ou des zones d'inhibition autour des disques permet de déterminer la sensibilité du micro-organisme présent dans l'échantillon ensemencé à chacun des agents antimicrobiens imprégnés dans chaque disque. Dû à la standardisation de la méthode de Kirby-Bauer, les résultats de ce procédé sont analysés en comparant le diamètre de chacune des zones d'inhibition avec les recommandations publiées par des organismes de régulation tels que le NCCLS (National Committee for Clinical Laboratory Standards) ou l'EUCAST (European Committee on Antimicrobial Susceptibility Testing). Les résultats sont ainsi couramment classifiés selon l'une des trois affirmations suivantes : sensible, intermédiaire ou résistant. Ces recommandations se traduisent donc par des seuils de références de susceptibilité associés correspondant à des tailles de zones d'inhibition pour chaque microorganisme vis-à-vis de chaque antibiotique.

Par « sensible », on entend qu'une croissance, voire une survie, des microorganismes présents dans l'échantillon, en présence de l'antibiotique est impossible, à partir d'une certaine concentration d'antibiotique. Par « intermédiaire », on entend qu'une croissance des microorganismes en présence de l'antibiotique est compromise, à partir d'une certaine concentration d'antibiotique. Par « résistant », on entend qu'une croissance des microorganismes en présence de l'antibiotique est possible, au moins jusqu'au seuil de toxicité de l'antibiotique pour le patient à traiter.

Une autre méthode de détection de sensibilité aux antibiotiques utilise un gradient d'antibiotique déposé sur un milieu gélosé. Pour cela, des bandelettes en papier ou en plastique sont imprégnées avec un gradient de concentration en antibiotique. Les bandelettes ainsi imbibées sont graduées afin d'indiquer les valeurs de concentration en antibiotique présentes le long de la bandelette. Une ou plusieurs bandelettes peuvent être placées sur une gélose Mueller-Hinton préalablement ensemencée de la même manière que la méthode précédente. Après incubation, une aire ovoïde d'inhibition de la croissance microbienne autour de chaque bandelette apparaît si le microorganisme présent dans l'échantillon est sensible à l'antibiotique compris dans la bandelette. Il est ainsi possible de déduire une concentration minimale inhibitrice (CMI) de la croissance microbienne. La concentration minimale de l'agent antimicrobien qui permet d'inhiber la croissance du microorganisme généralement retenue est ainsi la valeur de concentration lisible sur la graduation directement inférieure au point de contact entre la zone d'inhibition et le long bord de la bandelette. En d'autre termes, la CMI est la concentration visible à la limite de la zone d'inhibition, à la frontière entre la zone de croissance des microorganismes et la zone de non croissance. Plus particulièrement, la CMI est lisible en repérant le point où l'aire ovoïde de la zone d'inhibition coupe la bandelette et en repérant la graduation correspondante.

L'inconvénient de ces méthodes est qu'elles sont difficilement automatisables. En particulier, l'étape d'ensemencement par inondation requiert un mouvement circulaire et d'ondulation de la boîte par l'opérateur afin de bien répartir le dépôt de l'échantillon sur toute la surface de la gélose. Ce mouvement particulier requiert une certaine technicité de l'opérateur qui le réalise et est particulièrement difficile à reproduire par un automate. Ainsi, pour être mise en œuvre de façon automatisée, cette méthode nécessite des moyens de contrôle visuels du dépôt afin de s'assurer que la totalité de la surface de la gélose est recouverte par l'échantillon. L'automatisation du dépôt est également rendu complexe par les variations de viscosité entre les différents types d'échantillons liquides pouvant être employés, notamment entre des échantillons directement issus d'hémoculture et des échantillons remis en suspension. De plus, l'opération de retrait de l'excédent d'échantillon requiert également des moyens de pipettage et de détection précis de la surface de la gélose. Enfin, cette méthode nécessite des volumes importants d'échantillon, de l'ordre du millilitre, ce qui augmente le risque biologique lié à la manipulation de ces échantillons par l'opérateur.

L'automatisation du dépôt est également longue et fastidieuse avec un écouvillon ou une oese, notamment pour recouvrir la totalité de la surface du milieu de culture.

D'autre part, la lecture automatisée des zones d'inhibition est particulièrement difficile à partir des méthodes d'ensemencement traditionnelles, que ce soit pour la méthode des disques ou des bandelettes. Les zones d'inhibition présentent notamment des bords au contraste peu prononcé et pouvant être difficilement repérables par un système d'imagerie.

Enfin, un autre inconvénient est que ces méthodes nécessitent un volume d'échantillon important et contenant une forte biomasse, notamment une suspension calibrée à 0,5 Mc Farland de 1 mL. Cette quantité requiert souvent une étape préalable de pré-incubation de l'échantillon en présence d'un bouillon ou sur un milieu gélosé afin de pouvoir recueillir la quantité de colonies de microorganisme nécessaire. Cette étape préalable retarde ainsi le moment où un choix de traitement antibiotique adapté au type de microorganisme présent peut être effectué par le praticien. Il est ainsi fréquent que des antibiotiques à large spectre soient délivrés dans l'attente d'un résultat de sensibilité, ce choix s'avérant parfois inefficace et étant connu pour favoriser l'apparition de microorganismes résistants. Le document WO 00/55357 décrit un procédé de détection d'une présence au d'un absence d'une zone d'inhibition comprenant l'étalement d'un échantillon liquide susceptible de contenir des microorganismes sur la totalité d'un milieu de culture gélosé.

Un objectif de la présente invention est donc de proposer un procédé de détection d'une présence ou d'une absence d'au moins une première zone d'inhibition permettant d'utiliser une quantité réduite de biomasse à inoculer par rapport aux méthodes traditionnelles et, par conséquent, permettant de réduire le temps de pré-incubation nécessaire à la production de cette biomasse. Plus particulièrement, il est souhaitable de pouvoir caractériser de manière fiable et rapide une réponse des microorganismes présents ou susceptibles d'être présents dans l'échantillon à la présence de l'agent chimique. Cette réponse est préférentiellement obtenue à partir d'une culture inférieure à 6 heures et remise en suspension dans un faible volume de tampon.

Un second objectif de la présente invention est de proposer un procédé facilement automatisable, notamment comprenant des étapes de dépôt de l'échantillon et de lecture des zones d'inhibition rapides, fiables et répétables.

Pour cela, la présente invention concerne un procédé de détection d'une présence ou d'une absence d'au moins une première zone d'inhibition ledit procédé comportant les étapes consistant à :
a. Fournir un milieu de culture gélosé;
b. Fournir un échantillon contenant ou susceptible de contenir des microorganismes sous forme liquide ;
c. Déposer un volume de l'échantillon sous forme liquide selon une zone de dépôt s'étendant selon un axe à la surface du milieu de culture gélosé ;
d. Effectuer un dépôt d'une quantité déterminée d'un agent chimique à la surface du milieu de culture gélosé, ledit dépôt définissant une zone potentielle d'inhibition, l'axe de la zone de dépôt de l'échantillon intersectant la zone potentielle d'inhibition ;
e. Incuber ledit milieu de culture gélosé ;
f. Déterminer la présence ou l'absence de ladite première zone d'inhibition;
procédé dans lequel l'étape c) consiste :
- c1. A déposer un volume de l'échantillon sous forme liquide en ligne continue selon une zone de dépôt s'étendant selon un axe à la surface du milieu de culture gélosé ;
- ou c2. A déposer un volume de l'échantillon sous forme liquide en gouttelettes selon une zone de dépôt s'étendant selon un axe à la surface du milieu de culture.

La taille de la zone potentielle d'inhibition peut être définie comme la surface de la gélose, par exemple si la sensibilité du microorganisme à l'agent chimique est inconnue et/ou si le microorganisme est inconnu et/ou si la présence d'un microorganisme dans l'échantillon est inconnue.

Dans d'autre cas où le type de microorganisme, par exemple le genre, l'espèce ou la sous-espèce, est connu, cette information permet d'obtenir des informations sur sa sensibilité présumée à l'agent chimique à partir des recommandations d'organismes de régulation tels que le NCCLS (National Committee for Clinical Laboratory Standards) ou l'EUCAST (European Committee on Antimicrobial Susceptibility Testing). Ces recommandations présentent pour chaque couple formé d'un agent chimique et d'un microorganisme donné la taille de la zone potentielle d'inhibition obtenue après incubation pendant un temps donné selon que le microorganisme soit sensible, intermédiaire ou résistant à l'agent chimique.

Ainsi, selon un procédé avantageux de détection selon l'invention, l'échantillon contient une culture de microorganismes de type connu, l'aire de la zone potentielle d'inhibition étant alors définie par ledit type de microorganismes.

Les étapes de dépôts de l'échantillon pourront notamment être automatisées à l'aide d'un bras robotisé ou d'un pipetteur robotisé présentant un porte outil se déplaçant en translation selon trois degrés de liberté tel qu'un pipetteur Hamilton® Microlab Star. Les étapes de détermination de la présence des zones d'inhibition pourront notamment être réalisées à l'aide d'un dispositif de détection comprenant une source lumineuse et un moyen de captation de manière à capturer une image de l'échantillon déposé sur le milieu de culture puis en procédant à un examen visuel ou à un traitement automatisé de l'image ainsi obtenue.

L'avantage de l'invention est donc de pouvoir proposer un procédé facilement automatisable, notamment car l'étape de dépôt d'un volume de l'échantillon sous forme liquide est effectué selon une zone de dépôt s'étendant selon un axe à la surface du milieu de culture gélosé. Il est ainsi aisé pour un automate programmable de mettre en oeuvre cette étape de dépôt en suivant un axe dont les coordonnées sont préprogrammées ou sont déterminées par des méthodes classiques d'imagerie. D'autre part, la lecture du résultat du procédé de détection est également grandement facilitée du fait que l'échantillon, et donc la zone d'inhibition, sont localisés dans une zone plus restreinte du milieu de culture que dans un procédé classique par inondation ou par ensemencement à l'oese. L'axe de dépôt de l'échantillon sera ainsi rectiligne voire globalement rectiligne. Des variations de l'axe de dépôt utilisant des courbes, des portions de courbes ou des portions de droites consécutives pourront être envisagées si l'échantillon est déposé à proximité immédiate de la zone de dépôt de l'agent chimique. Notamment, dans le cas d'un support présentant un gradient de concentration d'agent chimique, tel qu'une bandelette, il peut être important de déposer l'échantillon selon un axe globalement rectiligne, préférentiellement adjacent au support de l'agent chimique, et ce quelle que soit la forme du support.

Selon un mode de réalisation :
- le dépôt du volume de l'échantillon est réalisé à l'aide d'une technique de dépôt prédéfinie apte, pour une biomasse de microorganismes donnée dans un volume d'échantillon sous forme liquide, à déposer ledit volume sur une surface maximale du milieu de culture gélosé de manière à obtenir une densité surfacique de microorganismes sensiblement homogène et supérieure à un seuil prédéfini ;
- l'échantillon est obtenu au moyen d'une pré-culture d'un échantillon brut, la pré-culture comprenant une phase d'isolement d'une souche de microorganisme suivie d'une phase d'incubation de ladite souche de manière à augmenter la biomasse de microorganismes, ladite biomasse dépendant de la durée d'incubation ; et
- la durée d'incubation de l'échantillon brut est choisie inférieure à 10 heures, avantageusement inférieure à 6 heures, et encore plus avantageusement comprise entre 3 heures et 6 heures, la surface du dépôt du volume d'échantillon à l'aide de la technique de dépôt prédéfinie étant choisie pour obtenir ladite densité.

En d'autres termes, il est recherché un dépôt d'une couche liquide de l'échantillon dans qui présente une concentration à la fois minimale en microorganisme et sensiblement homogène. Dans le cas contraire, une zone ne présentant pas ces caractéristiques de concentration pourrait présenter une pousse des microorganismes très faible, ce qui fausserait la mesure car une telle zone serait identifiée comme une zone d'inhibition. Or pour une technique de dépôt donnée, par exemple par inondation, au moyen d'un écouvillon ou d'une oese une biomasse minimale est nécessaire par unité de surface. Dans l'état de la technique, l'ensemencement consiste à déposer sur la totalité de la surface d'une boites de Pétri, ce qui nécessite une biomasse conséquente, et par conséquent un temps d'incubation important pour obtenir cette biomasse. Selon l'invention, il est visé une zone plus réduite de dépôt, la biomasse nécessaire étant donc également réduite, et partant de là le temps d'incubation pour obtenir cette biomasse réduite. Les inventeurs ont ainsi constater qu'un temps d'incubation inférieure à 6 heures permet d'obtenir la biomasse suffisante pour réaliser une mesure de CMI.

En particulier, la durée d'incubation nécessaire pour obtenir une biomasse suffisante pour un dépôt sur la totalité de la surface du milieu de culture gélosé avec ladite densité est supérieure à 20 heures, ce qui correspond au diamètre d'une boite de Pétri de 9 cm de diamètre ensemencée sur la totalité de sa surface.

Selon un premier mode de réalisation, l'étape c) du procédé de détection selon l'invention consiste à :
c. Déposer un volume de l'échantillon sous forme liquide en ligne continue selon une zone de dépôt s'étendant selon un axe à la surface du milieu de culture gélosé.

Le volume d'échantillon déposé sous forme d'une ligne continue peut présenter une concentration comprise entre 0,0005 Mc Farland et 0,5 Mc Farland. L'invention peut donc être applicable à des concentrations d'échantillons classiques : entre 0,5 Mc Farland et 0,1 Mc Farland, faibles : entre 0,1 Mc Farland et 0,01 Mc Farland, voire très faibles : entre 0,01 Mc Farland et 0,0005 Mc Farland. Ces concentrations pouvant être obtenues avec un temps minimal d'incubation voire sans incubation.

Selon un second mode de réalisation, l'étape c) du procédé de détection selon l'invention consiste à :
c. Déposer un volume de l'échantillon sous forme liquide en gouttelettes selon une zone de dépôt s'étendant selon un axe à la surface du milieu de culture gélosé.

Un dépôt de l'échantillon sous forme discrète peut ainsi être réalisé si les gouttelettes sont espacées d'une distance supérieure à leur diamètre une fois déposées. Avantageusement, les gouttelettes déposées sont espacées d'un pas prédéterminé, préférentiellement prédéterminé par l'aire de la zone potentielle d'inhibition et/ou le diamètre des gouttelettes déposées. Par exemple, les centres des gouttelettes déposées peuvent être espacées d'un pas millimétrique, préférentiellement d'un millimètre afin de pouvoir comparer rapidement le nombre de gouttes inhibées avec les recommandations des organismes de régulation. En effet ces recommandations éditent, pour un microorganisme et un agent chimique donné, la taille en millimètre de la zone d'inhibition mesurée selon que le microorganisme soit sensible, intermédiaire ou résistant. En comptant le nombre de gouttes inhibées et/ou le nombre de gouttes présentant une croissance, un résultat de sensibilité peut facilement être obtenu. Ainsi, dans une alternative de mise en œuvre du procédé, celui-ci comprend une étape consistant à déterminer le nombre de gouttelettes inhibées dans la zone potentielle d'inhibition et/ou le nombre de gouttelettes non inhibées dans la zone de dépôt de l'échantillon afin de détecter la présence d'une zone potentielle d'inhibition et d'en déduire la sensibilité du microorganisme présent dans l'échantillon à l'agent chimique.

Le volume de chaque gouttelette déposée est avantageusement compris entre 1nL et 10µL. Le procédé est donc applicable à des échantillons de faible à très faible volumes, notamment des échantillons issus de services hospitaliers pédiatriques. Le procédé a également pour avantage de ne pas être trop consommateur en volume d'échantillon ce qui permet d'effectuer d'autres analyses à partir d'un même échantillon.

Ainsi, les inventeurs ont estimé qu'un mode avantageux du procédé selon l'invention pouvait être mis en œuvre à partir d'une quantité de microorganismes contenue dans chaque goutte déposée comprise entre 1 microorganisme par goutte et 10⁶ microorganismes par goutte, préférentiellement de 10⁴ microorganismes par goutte. Ces ordres de concentration permettent donc d'analyser des échantillons sans incubation ou avec une durée limitée d'incubation permettant d'atteindre la concentration nécessaire.

Le dépôt de l'agent chimique est par exemple réduit à une gouttelette d'eau contenant l'agent chimique. Le procédé de détection selon l'invention est ainsi directement applicable aux tests utilisant des supports imprégnés d'agent chimique. Ainsi, l'étape d) peut avantageusement consister à :
d. Effectuer un dépôt d'un support imprégné d'une quantité déterminée d'un agent chimique à la surface du milieu de culture gélosé, ledit support définissant une zone potentielle d'inhibition, la zone de dépôt de l'échantillon intersectant la zone potentielle d'inhibition ;

Le dépôt de l'agent chimique est par exemple réalisé sur un support papier ou plastique imprégné. Ce support peut par exemple être un disque imprégné de l'agent chimique, le disque étant globalement conformé en une portion cylindrique de faible épaisseur. Le disque comporte une quantité en agent chimique qui est globalement homogène dans son volume.

Le support imprégné peut ainsi être un disque contenant une quantité déterminée d'agent chimique. Dans le cas d'un disque, le procédé de détection selon l'invention, peut comprendre une étape supplémentaire consistant à mesurer la distance entre le centre du disque et la première zone d'inhibition afin d'estimer la sensibilité des microorganismes contenus dans l'échantillon à l'agent chimique. Avantageusement, l'axe de dépôt de l'échantillon intersecte le centre du disque déposé sur le milieu. Préférentiellement, le procédé peut se poursuivre par une étape consistant à classer le microorganisme selon une classification à critères, par exemple : Sensible, Intermédiaire ou Résistant, à partir d'un abaque de sensibilité correspondant au microorganisme présent dans l'échantillon et à l'agent chimique. Cet abaque peut notamment être obtenu de façon expérimentale par apprentissage ou à partir de recommandations d'organismes de régulation.

Selon un mode particulier de réalisation de l'invention, l'étape d) consiste à :
d) Effectuer au moins deux dépôts d'une quantité déterminée d'un agent chimique à la surface du milieu de culture gélosé, lesdits dépôts définissant chacun une zone potentielle d'inhibition, l'axe de la zone de dépôt de l'échantillon intersectant toutes les zones potentielles d'inhibition ;

Ce mode particulier permet notamment d'étudier les effets de synergie entre plusieurs agent chimiques, notamment imprégnés sur des disques. Par exemple, deux disques comprenant deux agents différents peuvent être déposés sur un milieu de culture, l'axe de dépôt de l'échantillon intersectant le centre de ces deux disques. Dans un autre exemple, quatre disques comprenant quatre agents différents sont déposés sur un milieu de culture, l'axe de dépôt de l'échantillon intersectant le centre de ces quatre disques de sorte à suivre les bords d'un rectangle.

Le support imprégné est par exemple encore une bandelette longiligne, de conformation globalement rectangulaire. La bandelette comporte par exemple une concentration en agent chimique qui suit un gradient croissant de concentration d'un petit bord à un petit bord opposé de la bandelette. Ainsi, dans une mise en œuvre alternative du procédé de détection selon l'invention, le support imprégné est une bandelette contenant un gradient de concentration d'agent chimique, le dépôt du volume de l'échantillon sous forme liquide étant effectué parallèlement et préférentiellement de façon adjacente à au moins un grand bord de ladite bandelette. Par adjacent on entend que le dépôt est effectué au plus proche du grand bord de la bandelette sans pour autant que l'échantillon liquide déposé soit en contact avec la bandelette. En effet, il est peu souhaitable que l'échantillon soit en contact direct avec la bandelette, ceci pouvant mouiller la bandelette et ainsi modifier localement la diffusion de l'agent chimique dans et sur la gélose.

Avantageusement, le procédé comprend une étape supplémentaire consistant à :
- localiser une frontière entre la première zone d'inhibition et la zone de croissance des microorganismes
- déterminer une concentration minimale inhibitrice de l'agent chimique à partir de la localisation de ladite frontière

L'étape de localisation d'une frontière entre la première zone d'inhibition et la zone de croissance des microorganismes peut être réalisée de manière visuelle ou en captant une image du milieu de culture suite à l'étape d'incubation par un moyen d'acquisition, puis en recherchant une droite ou un arc présent à l'intersection entre la zone de croissance et la zone d'inhibition de l'échantillon. Selon des techniques connues de l'homme du métier, la frontière entre la zone d'inhibition et la zone de croissance pourra être obtenue à partir d'une image ou d'une combinaison d'images. Cette ou ces images permettant avantageusement de visualiser à la fois les graduations présentes sur la bandelette et la frontière entre la zone d'inhibition et de croissance. Une technique classique consiste à obtenir une image en vue de dessus des graduations de la bandelette et à la combiner avec une image en transmission du milieu de culture.

Une méthode classique consiste, à partir d'une image numérique, dans la définition d'un repère cartésien, typiquement dont l'axe des abscisses est défini comme l'axe principale de la bandelette. Il est alors possible de localiser une marque définie de la bandelette, typiquement un texte connu tel qu'une graduation. Par exemple, les caractères « 256 » correspondant à la concentration à 256µg.ml⁻¹ d'agent chimique peuvent être localisés dans le repère. La marque définie est susceptible d'être constituée d'une indication autre et relativement quelconque. La marque définie peut également correspondre à un des petits bords de la bandelette. La marque définie comporte alors des coordonnées cartésiennes dans le repère cartésien. De la même manière, les grands bords de la bandelette peuvent aisément être reconnus par des moyens classiques de traitement d'image afin d'obtenir leurs coordonnées dans le repère.

Par la suite, l'image est traitée afin de rechercher la frontière entre la zone d'inhibition et de croissance. Cette étape peut éventuellement comprendre une opération de lissage pour homogénéiser l'image. Une telle opération de lissage est par exemple réalisée à partir d'un filtrage gaussien. De préférence, l'opération de lissage est réalisée à plusieurs reprises, sept fois notamment. Cette étape peut éventuellement comprendre une opération de dilatation de la dynamique de l'intensité de pixels de l'image pour former un histogramme de contraste de l'image, le contraste étant à considérer entre pixels sombres et pixels clairs de l'image. Il en résulte une détermination d'une dynamique utile de l'image. Cette étape peut alors comprendre une opération de seuillage de l'image qui comprend par exemple une détection d'un seuil et la détermination d'un contour à partir d'une binarisation de l'image. A partir de ce contour, il peut alors être extrapolé une droite ou un arc représentant la frontière entre la zone de croissance et d'inhibition, cette droite ou cet arc étant recherchés dans une zone de pixel proche du grand bord de la bandelette, par exemple à moins d'un centimètre du grand bord.

A partir de cette frontière, une opération d'estimation d'une concentration minimale d'inhibition, peut être réalisée. Une méthode possible consiste à déterminer les coordonnées en abscisse du point de croisement entre la droite ou l'arc obtenu et le grand bord de la bandelette. Alternativement une méthode peut consister à déterminer les coordonnées en abscisse du point de croisement entre la droite ou l'arc obtenu et l'axe des abscisses du repère cartésien. Une fois l'abscisse obtenue, en relation avec l'origine du repère et la longueur connue de la bandelette, la concentration minimale d'inhibition peut être déterminée. Dans certains cas, il est possible que le milieu de culture ne comprenne que des gouttelettes totalement inhibées ou totalement en croissance, c'est à dire où aucune frontière entre une zone de croissance et d'inhibition n'est visible ou déterminable. Dans ce cas, la valeur de CMI pourra directement être obtenue par comptage des gouttelettes en relation avec le pas de dépôt, ou à partir de la localisation des gouttelettes dans le repère, notamment de la localisation de la première gouttelette non inhibée selon le gradient croissant de concentration en agent chimique. Alternativement, la valeur de CMI pourra directement être obtenue à partir de la localisation de la dernière gouttelette inhibée selon le gradient croissant de concentration en agent chimique.

On entend par milieu de culture un milieu gélosé, présentant une couche d'agar ou analogue. Les milieux de culture se retrouvent communément contenus dans une boîte de Pétri ou sous forme déshydratée appliqués sur un support, généralement un film. De façon non limitative, d'autres types de milieu de culture peuvent être utilisés tels que des milieux de culture sur support fibreux ou encore des milieux sur support papier.

L'agent chimique est notamment un antibiotique, un antifongique, un antimycobactérien ou un composé analogue.

Par susceptible de contenir, on entend que la présence de microorganismes dans l'échantillon peut être suspectée à partir du type de prélèvement ou encore des symptômes du patient ou de l'animal sur lequel l'échantillon est prélevé. Cependant le type de microorganisme susceptible d'être contenu dans l'échantillon n'est alors pas connu. Dans le cas de la recherche de mammite chez la vache par exemple, il peut être plus efficace de directement déterminer une concentration minimale inhibitrice par des agents chimiques classiques avant de connaître l'identification du type de microorganisme en présence dans l'échantillon prélevé directement sur le pis de la vache. Un traitement efficace de l'infection par le microorganisme peut alors être prescrit.

D'autres caractéristiques et avantages de la présente invention apparaîtront à la lecture de la description qui va en être faite d'exemples de réalisation, en relation avec les figures des planches annexées, dans lesquelles :
- La figure 1 est une vue en coupe d'une boîte de Pétri utilisée pour la mise en œuvre d'un procédé de détection de la présente invention.
- La figure 2 est une vue schématique du procédé de détection de la présente invention.
- La figure 3 est une illustration schématique de séquences du procédé de détection de la présente invention.
- Les figures 4a à 4f illustrent un premier mode de réalisation du procédé de détection selon la présente invention.
- La figure 5 est une illustration schématique d'un dispositif de captation d'image par ombroscopie.
- Les figures 6a et 6b illustrent des images prises en vue de dessus d'une boîte de Pétri utilisée pour la réalisation d'un mode de réalisation du procédé de détection selon la présente invention en comparaison avec une technique classique.
- Les figures 7a à 7f illustrent un second mode de réalisation du procédé de détection selon la présente invention.
- La figure 8a illustre en vue schématique un second exemple de mise en œuvre de l'invention
- Les figures 8b, 8c et 8d illustrent le second exemple de mise en œuvre de l'invention.
- Les figures 9a à 9d illustrent le second exemple de mise en œuvre de l'invention pour quatre antibiotiques différents
- Les figures 10a, 10b illustrent une partie des bandelettes et des gouttelettes proches de celles-ci après 5 heures d'incubation d'un échantillon d'*Escherichia coli* ATCC 35218 à 0,5 Mc Farland et 0,01 Mc Farland en présence d'ampicilline/sulbactam.
- Les figures 11a, 11b illustrent une troisième méthode de mise en œuvre de l'invention
- La figure 12 illustre un examen visuel après 6h30 d'incubation d'un milieu ensemencé selon la troisième méthode de mise en œuvre de l'invention
- La figure 13 illustre un exemple d'outil de prélèvement pouvant être utilisé selon la troisième méthode de mise en œuvre de l'invention

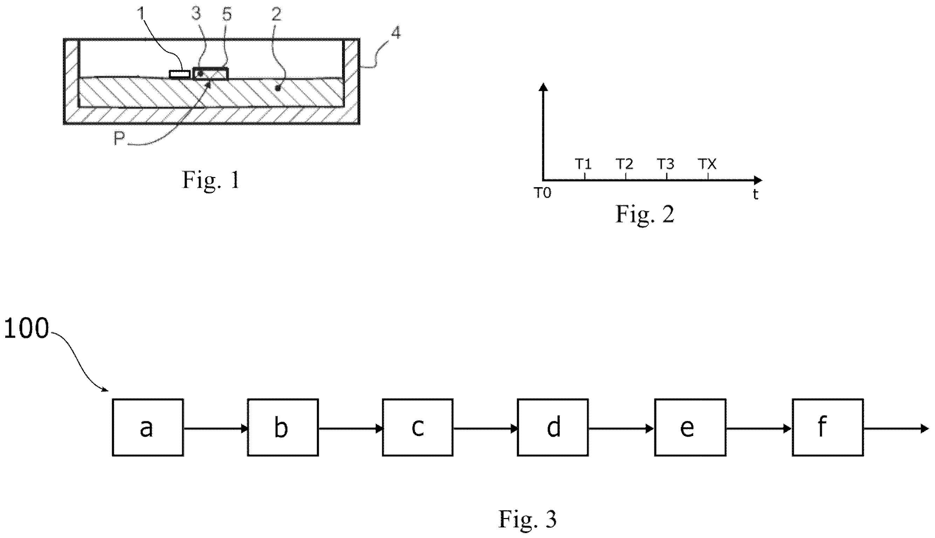

En se reportant sur les figures 1 et 3, dans le domaine médical et/ou pharmaceutique, il est fréquent d'avoir à utiliser un procédé de détection 100 d'une présence ou d'une absence d'une zone d'inhibition sur un milieu de culture 2 d'un échantillon 1 en présence d'un agent chimique 5 imbibé sur un support 3. Le milieu de culture 2 est contenu dans une boite de Pétri 4, par exemple une boite de Pétri d'une diamètre supérieur ou égal à 9 cm, et reçoit l'échantillon 1 contenant ou susceptible de contenir les microorganismes, ainsi que l'agent chimique 5 qui est apte à inhiber un développement de certains microorganismes. Les microorganismes sont choisis indifféremment parmi les bactéries, les levures ou les champignons. Alternativement, le procédé selon la présente invention peut être appliqué à des cellules végétales ou animales. Le milieu de culture 2 est préférentiellement une gélose, une couche d'agar ou analogue. Le milieu de culture 2 peut également être un milieu de culture déshydraté sur support papier ou support fibreux. Le milieu est alors réhydraté par l'échantillon. L'agent chimique 5 est notamment un antibiotique, un antifongique, un antimycobactérien ou un composé analogue.

Plus particulièrement, il est souhaitable de pouvoir caractériser de manière fiable et rapide une réponse de l'échantillon contenant ou susceptible de contenir les microorganismes 1 à la présence de l'agent chimique 5, une telle réponse étant classifiée couramment selon l'une des trois affirmations suivantes : sensible, intermédiaire ou résistant. Il peut également être souhaitable d'obtenir une valeur de concentration minimale inhibitrice d'agent chimique pouvant inhiber la croissance de microorganismes présents dans l'échantillon.

Un tel procédé de détection 100 trouve notamment des applications fréquentes dans le domaine des diagnostics médicaux, pharmaceutiques et/ou vétérinaires mis en œuvre pour la détection d'une pathologie chez un patient ou un animal. Il en résulte qu'un tel procédé de détection 100 est souhaité fiable dans le sens où la nature de la réponse susvisée des microorganismes contenus ou susceptibles d'être contenus dans l'échantillon 1 à l'agent chimique 5 est voulue certaine, sans doute et sans ambiguïté. Il en résulte aussi qu'un tel procédé de détection 100, dont des séquences successives sont illustrées sur les figures 2 et 3, est souhaité rapide avec un temps de réponse, qui s'écoule entre un temps initial T0 auquel l'échantillon est mis au contact de l'agent chimique 5 et un temps de détection TX auquel ladite réponse fiable est obtenue, qui est souhaité le plus court possible, et est notamment inférieur à huit heures. Il en résulte aussi qu'il est souhaitable qu'un tel procédé de détection 100 comporte une répétabilité idoine. De tels buts sont avantageusement atteints à partir de la mise en œuvre du procédé de détection 100 de la présente invention.

Dans sa généralité, et en se reportant sur la figure 3, le procédé de détection 100 de la présente invention comprend une étape a) de fourniture d'un milieu de culture gélosé 2, une étape b) de fourniture d'un échantillon 1 contenant ou susceptible de contenir des microorganismes sous forme liquide. Par exemple un échantillon brut directement prélevé sur le patient. Dans ce cas-là, l'échantillon brut peut subir une phase de préculture permettant un isolement de souches de microorganismes en présence, par exemple dans le cas d'un échantillon comprenant une diversité de microorganismes, et une incubation des microorganismes sélectionnés et isolés. L'échantillon 1 est par exemple encore un échantillon préparé, notamment filtré, centrifugé et/ou purifié de manière similaire. L'échantillon 1 peut présenter une biomasse de microorganismes qui est suffisante pour être analysée valablement, tel qu'un étalon de concentration comprise entre 0,0005 Mc Farland et 0,5 Mc Farland. L'échantillon 1 est par exemple de l'urine, du sang, du liquide céphalorachidien ou un liquide biologique analogue.

Un volume de l'échantillon sous forme liquide est ensuite déposé dans l'étape c) selon une zone de dépôt s'étendant selon un axe à la surface du milieu de culture gélosé. L'échantillon peut être déposé en gouttelettes, notamment à l'aide d'une pipette manuelle ou d'un automate pipetteur. Les gouttelettes peuvent avantageusement présenter un volume identique et être espacées d'un pas P prédéterminé, P étant la distance entre les centres de deux gouttelettes consécutives. Dans le cas où le diamètre des gouttelettes une fois déposées est supérieur à la valeur du pas P, les gouttelettes forment alors un dépôt liquide en ligne continue.

L'échantillon peut également être déposé par un écouvillon, notamment un écouvillon floqué ou fibreux trempé dans un volume de l'échantillon puis déplacé au contact du milieu gélosé selon un axe. Alternativement, un dispositif de pipettage comprenant des moyens de filtration tel que décrit dans la demande internationale publiée sous le numéro WO2012/083150 A2 peut être utilisé pour pipeter et filtrer un volume d'échantillon et le déposer par frottement au contact de la surface du milieu de culture gélosé. Le déplacement de l'écouvillon ou du dispositif de pipettage comprenant des moyens de filtration décrits ci-dessus au contact de la gélose permet ainsi de former un dépôt de l'échantillon en ligne continue, cette ligne s'étendant selon un axe.

Le procédé se poursuit dans l'étape d) par le dépôt d'une quantité déterminée d'un agent chimique à la surface du milieu de culture gélosé, ledit dépôt définissant une zone potentielle d'inhibition, l'axe de la zone de dépôt de l'échantillon intersectant la zone potentielle d'inhibition. Alternativement, ce dépôt peut être effectué avant le dépôt de l'échantillon. L'instant initial T0 est considéré comme le moment ou l'échantillon et l'agent chimique sont mis en contact à la surface du milieu de culture.

Le procédé se poursuit dans l'étape e) par l'incubation du milieu de culture gélosé.

Le procédé se poursuit dans l'étape f) qui consiste à déterminer à un temps Tx d'incubation la présence ou l'absence de ladite première zone d'inhibition de l'échantillon autour de la zone de dépôt de l'agent chimique, dans la zone potentielle d'inhibition.

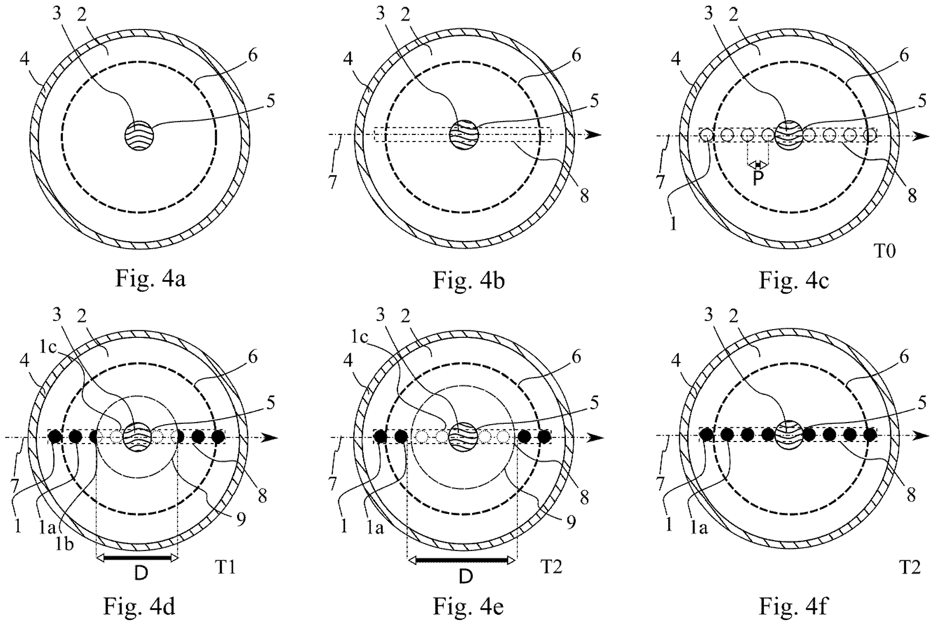

Un premier exemple de mise en œuvre de l'invention va être détaillé selon les figures 4a à 4f illustrant des boites de Pétri 4 (par exemple des boites de Pétri d'un diamètre supérieur ou égal à 9 cm) en vue de dessus.

En se reportant sur la figure 4a, le premier exemple de mise en œuvre du procédé de détection selon la présente invention comprend une étape de fourniture d'un milieu de culture gélosé 2 dans une boite de Pétri 4.

Le dépôt d'un disque 3 imprégné d'une quantité déterminée d'un agent chimique 5 est effectué à la surface du milieu de culture gélosé, ledit dépôt définissant une zone potentielle d'inhibition circulaire 6 autour du disque.

Selon la figure 4b, un axe 7 de dépôt intersectant la zone potentielle d'inhibition 6 et le centre du disque 3 est défini. Cet axe définit également une zone de dépôt 8 de l'échantillon 1 globalement rectangulaire et équitablement répartie de part et d'autre de l'axe de dépôt.

Selon la figure 4c, un échantillon liquide 1 est déposé à T0, sous forme de multiples gouttelettes, à la surface du milieu de culture 2, dans la zone de dépôt 8 et selon l'axe 7. Les gouttelettes sont espacées d'un pas P, correspondant à la distance entre le centre de deux gouttelettes consécutives. L'incubation du milieu de culture débute alors.

Selon la figure 4d, à l'instant T1 après un temps d'incubation, la présence d'une première zone d'inhibition 9 de l'échantillon 1 est déterminée, par exemple par analyse visuelle. En effet, certaines gouttelettes présentent une croissance bactérienne 1a, tandis que d'autres ne présentent aucune croissance bactérienne, 1c. A l'intersection entre la première zone d'inhibition 9 et la zone de dépôt de l'échantillon 8, certains gouttelettes présentent une partie en croissance et une partie inhibée 1b. Il est ainsi possible à cet instant de démontrer la présence de microorganismes dans l'échantillon ainsi que l'inhibition de la croissance de ces microorganismes en présence de l'agent chimique 5. La zone d'inhibition 9 présente un diamètre D pouvant notamment être mesuré par un pied à coulisse.

Selon la figure 4e, à l'instant T2 après un temps plus important d'incubation, la surface de la première zone d'inhibition 9 et son diamètre D sont stables et ne varient plus. Il est ainsi possible à cet instant de mesurer de façon fiable la taille de la zone d'inhibition. Cette détermination est particulièrement aisée dans le cas de la figure 4e où certaines gouttelettes présentent une croissance bactérienne 1a, tandis que d'autres ne présentent aucune croissance bactérienne, 1c, une simple opération de comptage, en rapport avec le pas P de dépôt entre chaque goutte permet donc de déterminer la sensibilité du microorganisme à l'agent chimique. D'autre part, en obtenant une identification du microorganisme en présence et en comparant la taille de la zone d'inhibition avec les recommandations des organismes de régulation pour le couple agent chimique 5, microorganisme en présence, la classification de la souche comme « sensible », « intermédiaire » ou « résistante » est également possible.

Selon la figure 4f, il est probable qu'a un instant T2, la première zone d'inhibition 9 ne soit plus visible ou fortement réduite et que certaines voire la totalité des gouttelettes présentent une croissance bactérienne la. Il est ainsi possible à cet instant de déterminer que le type de microorganisme présent dans l'échantillon 1 est résistant à l'agent chimique 5. Il est également possible qu'aucune zone d'inhibition ne soit apparente et ce quelle que soit la durée d'incubation, démontrant la résistance du microorganisme à l'agent chimique.

Afin de mettre en œuvre un procédé selon l'invention, il peut par exemple être employé un dispositif de captation comprenant un moyen de captation et une source lumineuse de manière à capturer une image de l'échantillon déposé sur le milieu de culture en présence d'un agent chimique.

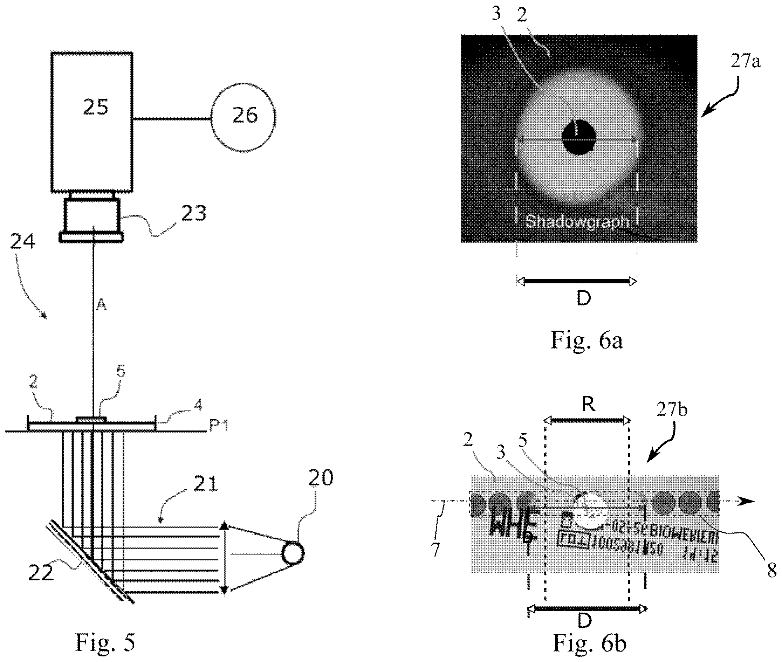

Un exemple de dispositif de captation 24 par ombroscopie est illustré sur la figure 5. Ce dispositif comprend un moyen de captation 25. Le moyen de captation 25 comporte un axe de captation A qui est préférentiellement ménagé orthogonalement par rapport à un premier plan P1 selon lequel est étendu le milieu de culture 2. Le moyen de captation 25 surplombe avantageusement la boîte de Pétri 4 de manière à prendre une vue de dessus du milieu de culture 2. Le moyen de captation est par exemple une caméra CCD, notamment de type Basler piA2400 - 17 gm, qui est équipée d'un objectif télécentrique 23. La source lumineuse 20 est préférentiellement un illuminateur collimaté apte à produire des rayons lumineux 21 parallèles entre eux qui atteignent orthogonalement le milieu de culture 2 après avoir été réfléchis par un miroir 22. La source lumineuse 20 peut comprendre une pluralité de diodes comportant une gamme d'émission indifféremment dans le rouge, le vert, le bleu et le blanc. La source lumineuse 20 est par exemple du type Opto Engineering - LTCL 048 - W. L'objectif télécentrique 23 est notamment de type Opto Engineering - TC23 048, comportant un champ focal de 46x38,5 mm et une distance de travail de 134,6 mm. Avantageusement, le dispositif 24 peut comprendre des moyens de calcul 26 comprenant par exemple des moyens d'analyse et de traitement d'images, les moyens de calcul 26 étant constitutifs d'un processeur que comprend le dispositif de captation 24. Avantageusement, le dispositif 24 peut comprendre une ou d'autres source(s) lumineuse(s) (non representée(s)) disposée(s) au-dessus du milieu de culture et dirigée(s) vers le milieu de culture. Ces sources permettent par exemple d'éclairer de façon plus optimale la partie imprimée du support imprégné d'agent chimique, notamment de manière à localiser plus facilement une marque définie du support tel qu'un ou des caractère(s) imprimé(s) sur le support.

Les figures 6a et 6b, permettent de comparer les résultats d'une méthode traditionnelle de détection d'une présence ou d'une absence d'au moins une première zone d'inhibition avec la méthode selon l'invention. Pour la réalisation de cette comparaison, deux géloses Mueller Hinton E (bioMérieux Ref. 413822) sont ensemencées avec un inoculum issu d'une culture de *Staphylococcus aureus* ATCC 25923 (American Type Culture Collection) à la concentration de 0,5 Mc Farland. La première gélose est ensemencée par inondation par un volume de 1mL environ, tandis qu'à la surface de la seconde gélose sont déposées dix gouttelettes de 3µL séparées d'un pas de 5 mm selon un axe 7 dans une zone de dépôt 8. Un disque 3 imbibé contenant 10µg d'ampicilline est également déposé sur chacune des géloses. Sur la figure 6b, le disque est déposé de façon à intersecter l'axe 7 de dépôt des gouttelettes et la zone 8 de dépôt de l'échantillon. Les deux géloses sont ensuite incubées à 37°C pendant 6 heures 30 minutes. Suite à ce temps d'incubation, une image en vue de dessus de chaque gélose est captée à l'aide d'un dispositif 24 de captation par ombroscopie tel que précédemment décrit et permettant d'obtenir les images 27a et 27b des figures 6a et 6b. Au temps de captation des images 27a et 27b, il est ainsi possible d'observer un diamètre D, correspondant à une zone d'inhibition, similaire entre les méthodes et supérieur au seuil de référence R défini par l'EUCAST. En effet, le diamètre D de la zone d'inhibition est de 22mm, le seuil de référence R entre sensible et résistant défini par l'EUCAST étant égal à 18mm. La méthode selon l'invention permet donc d'utiliser un volume minimal d'échantillon tout en obtenant un résultat identique de sensibilité à un agent chimique. De nombreuses techniques connues de l'homme du métier peuvent être employées afin de déterminer le diamètre D de façon automatisée. Par exemple, une méthode peut consister à repérer l'axe de dépôt des gouttes ainsi que le centre du disque à partir d'une image numérique du milieu de culture. Par la suite, il est possible d'extraire un profil d'intensité des pixels de l'image selon un axe passant par le centre du disque et selon ou parallèlement à l'axe de dépôt des gouttelettes. A partir de ce profil d'intensité, on recherche alors les transitions de contraste les plus importantes de part et d'autre du disque. Ces transitions peuvent notamment être recherchées en recherchant la position dans le profil du premier front montant, ou la valeur maximale de la dérivée première du profil. Les deux positions obtenues de part et d'autre du disque correspondent alors au diamètre D de la zone d'inhibition recherché.

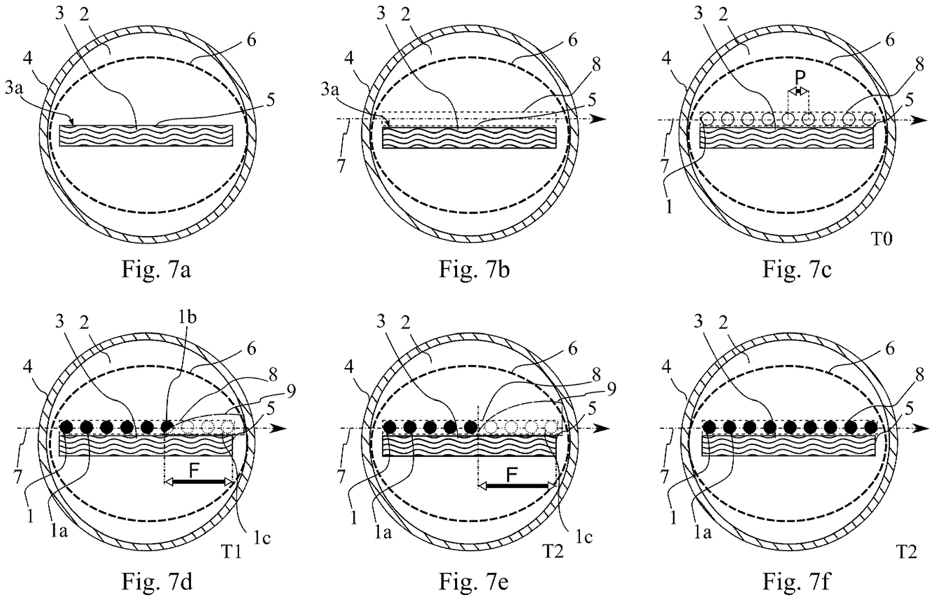

Un second exemple de mise en œuvre de l'invention va être détaillé selon les figures 7a à 7f.

En se reportant sur la figure 7a, le second exemple de mise en œuvre du procédé de détection selon la présente invention comprend une étape de fourniture d'un milieu de culture gélosé 2 dans une boite de Pétri 4, par exemple une boite de Pétri d'un diamètre supérieur ou égal à 9 cm.

Le dépôt d'une bandelette 3 imprégnée d'une quantité déterminée d'un agent chimique 5 est effectué à la surface du milieu de culture gélosé, ledit dépôt définissant une zone potentielle d'inhibition ovoïde 6 autour de la bandelette.

Selon la figure 7b, un axe 7 de dépôt intersectant la zone potentielle d'inhibition 6 et parallèle à un des grands bords 3a de la bandelette 3 est défini. Cet axe définit également une zone de dépôt 8 de l'échantillon globalement rectangulaire et équitablement répartie de part et d'autre de l'axe de dépôt.

Selon la figure 7c, un échantillon liquide 1 est déposé à T0, sous forme de multiples gouttelettes, à la surface du milieu de culture 2, dans la zone de dépôt et selon l'axe 7. Les gouttelettes sont espacées d'un pas P, correspondant à la distance entre le centre de deux gouttelettes consécutives. L'incubation du milieu de culture débute alors.

Selon la figure 7d, à l'instant T1 après un temps d'incubation, la présence d'une première zone d'inhibition 9 de l'échantillon 1 est déterminée, par exemple par analyse visuelle. En effet, certaines gouttelettes présentent une croissance bactérienne 1a, tandis que d'autres ne présentent aucune croissance bactérienne, 1c. A l'intersection entre la première zone d'inhibition 9 et la zone de dépôt de l'échantillon 8, certains gouttelettes présentent une partie en croissance et une partie inhibée 1b. Il est ainsi possible à cet instant de démontrer la présence de microorganismes dans l'échantillon ainsi que l'inhibition de la croissance de ces microorganismes en présence de l'agent chimique 5.

Selon la figure 7e, à l'instant T2 après un temps plus important d'incubation, la surface de la première zone d'inhibition 9 est stable et ne varie plus. Il est ainsi possible à cet instant de mesurer de façon fiable la taille de la zone d'inhibition et d'en déduire la valeur F correspondant à la concentration minimale inhibitrice de l'agent chimique. Cette valeur F correspond à la concentration d'agent chimique imbibé sur la bandelette à l'intersection entre la zone de pousse et d'inhibition de l'échantillon.

Dans le cas où le milieu ne présente que des gouttelettes avec une croissance bactérienne 1a, ou sans croissance bactérienne 1c, la valeur F correspond à la valeur intermédiaire obtenue en traçant une perpendiculaire à l'axe 7 entre la dernière gouttelette inhibée et la première gouttelette en croissance dans le sens des concentrations croissantes de la bandelette et en observant la valeur de concentration en agent chimique correspondante sur la bandelette.

Dans le cas où le milieu présente une gouttelette avec une partie en croissance et une partie inhibée 1b, la valeur F peut notamment être obtenue en recherchant un arc ou un droite à la frontière entre la partie en croissance et la partie inhibée ; en recherchant l'intersection de cet arc ou de cette droite avec le grand bord 3a de la bandelette et en observant la valeur de concentration en agent chimique à cette intersection. Alternativement, les gouttelettes présentant une partie en croissance et une partie inhibée 1b peuvent être ignorées afin de rechercher la première valeur de concentration plus importante inhibant complètement la croissance d'une gouttelette.

Selon la figure 7f, il est possible qu'à un instant T2 la première zone d'inhibition 9 ne soit plus visible ou réduite et que certaines voire la totalité des gouttelettes présentent une croissance bactérienne la. Il est ainsi possible à cet instant de déterminer que le microorganisme présent dans l'échantillon 2 est résistant à l'agent chimique 5. Il est également possible qu'aucune zone d'inhibition ne soit apparente et ce quelle que soit la durée d'incubation, démontrant la résistance du microorganisme à l'agent chimique.

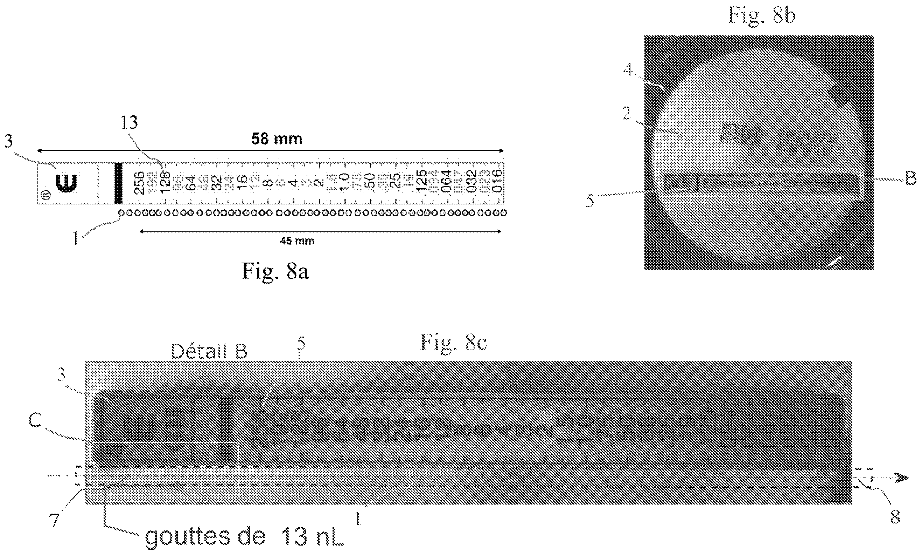

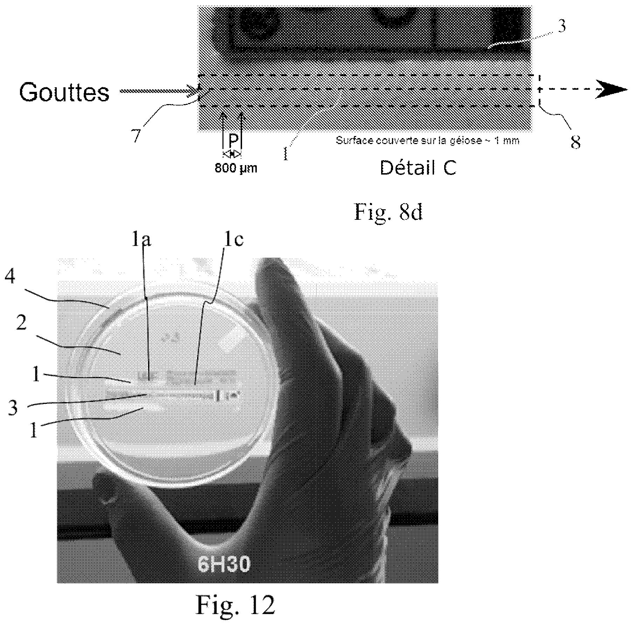

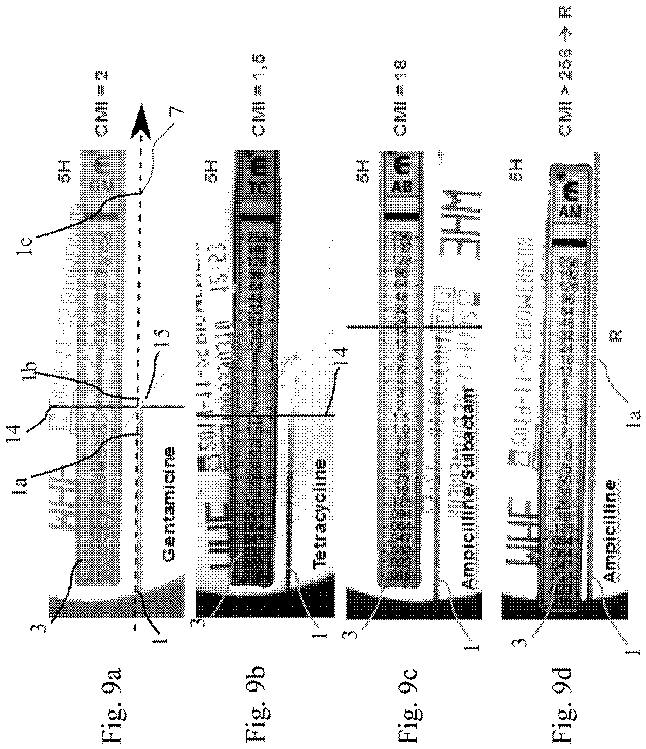

Les figures 8a à 8d et 9a à 9d illustrent ce second exemple de mise en œuvre de l'invention. Selon la figure 8a, un échantillon 1 est déposé sous forme d'une série de gouttelettes, selon un axe parallèle au grand bord d'une bandelette 3 contenant un gradient de concentration d'agent chimique. La bandelette 3 comprend des graduations de concentrations d'agent chimique 13 permettant de déduire une concentration minimale inhibitrice d'agent chimique. Un exemple de telle bandelette est commercialisé par la demanderesse sous la marque de commerce Etest®. Selon la figure 8b, une bandelette 3 contenant un agent chimique 5 est déposée sur un milieu de culture 2 contenu dans une boite de Pétri 4 (par exemple une boite de Pétri d'un diamètre supérieur ou égal à 9 cm). Le détail B de la figure 8b est visible sur la figure 8c. Un échantillon 1 est déposé sous forme d'une série de gouttelettes, dans une zone de dépôt 8 selon un axe 7 parallèle au grand bord de la bandelette 3. Dans cet exemple, des gouttes de 13 nanolitres sont déposées par un pipetteur Pipejet P9 Nanodispenser commercialisé par la société biofluidix et monté sur un bras robotisé. Le détail C de la figure 8c est visible sur la figure 8d. 50 gouttelettes sont ainsi déposées selon l'axe 7 avec un pas P de 800µm. Chaque gouttelettes couvre une surface d'environ 1mm².

Selon une première expérience de cet exemple, un inoculum d'une souche d'*Escherichia coli* ATCC 35218 (American Type Culture Collection) présentant une concentration de 0,5 Mc Farland est ainsi déposé en gouttelettes le long de quatre bandelettes Etest® (bioMérieux), chacune déposée sur un milieu de culture gélosé Mueller Hinton E (bioMérieux). Les bandelettes contiennent respectivement un gradient de concentration de : gentamicine, tétracycline, ampicilline/sulbactam, ampicilline. De la même manière, un inoculum d'une souche d'*Escherichia coli* ATCC 25922 présentant une concentration de 0,5 Mc Farland est déposé en gouttelettes le long de quatre bandelettes Etest® (bioMérieux), chacune déposée sur un milieu de culture gélosé Mueller Hinton E (bioMérieux). Les bandelettes contiennent également un gradient de concentration de : gentamicine, tétracycline, ampicilline/sulbactam, ampicilline.

Cinquante gouttelettes, d'un volume de 13 nanolitres chacune, et espacées de 800 µm sont déposées le long de chaque bandelette. En fonction de la concentration de 0,5 Mc Farland, il est ainsi estimé qu'environ 2000 bactéries sont présentes dans chaque gouttelette. Les huit milieux de cultures ainsi préparés sont ensuite incubés et surveillés périodiquement afin de déterminer la présence ou l'absence d'une zone d'inhibition et/ou de croissance des microorganismes.

Les figures 9a à 9d illustrent en vue de dessus, les quatre milieux où l'échantillon d'*Escherichia coli* ATCC 35218 a été déposé. Les figures 9a à 9d sont obtenues à l'aide d'un dispositif de captation par ombroscopie tel que présenté en relation avec la figure 5. La figure 9a présente une vue après 5 heures d'incubation de l'échantillon en présence de gentamicine. L'échantillon présente alors des gouttelettes en croissance 1a, des gouttelettes où la croissance est inhibée 1c et au moins une gouttelette en partie inhibée 1b. A partir de techniques classiques d'analyse d'image de la gouttelette en partie inhibée 1b, il est obtenu une droite 14 perpendiculaire au grand bord de la bandelette et délimitant la frontière de la zone de croissance des microorganismes et de la zone d'inhibition. Une valeur de concentration minimale inhibitrice est alors obtenue à l'intersection entre la droite 14 et le grand bord de la bandelette. Une concentration minimale inhibitrice (CMI) de 2µg/ml est alors déterminée.

De façon avantageuse, une droite ou un arc 15 délimitant la frontière de la zone de croissance des microorganismes et de la zone d'inhibition peut être recherchée, cette droite n'étant pas nécessairement perpendiculaire au grand bord et restituant ainsi plus fidèlement ladite frontière. Cette droite ou cet arc correspond à une portion de l'aire ovoïde d'inhibition observée avec des méthodes classiques d'ensemencement et permet donc une meilleure estimation de la CMI. Dans le cas où une droite ou un arc 15 est recherchée, la valeur de concentration minimale inhibitrice peut être obtenue en traçant une perpendiculaire au grand bord de la bandelette croisant le point d'intersection entre la droite 15 et l'axe de dépôt de l'échantillon 7, puis en recherchant la graduation correspondante à cette perpendiculaire sur la bandelette

La figure 9b présente une vue après 5 heures d'incubation de l'échantillon en présence de tétracycline. Un procédé similaire est répété et permet d'estimer une concentration minimale inhibitrice de 1,5µg/ml. La figure 9c présente une vue après 5 heures d'incubation de l'échantillon en présence d'ampicilline/sulbactam. Un procédé similaire est répété et permet d'estimer une concentration minimale inhibitrice de 18µg/ml. La figure 9d présente une vue après 5 heures d'incubation de l'échantillon en présence d'ampicilline. Sur cette figure, il n'est observé que des gouttelettes en croissance 1a, démontrant la résistance du microorganisme à l'ampicilline, la valeur observée de concentration minimale inhibitrice est alors supérieure à 256µg/ml.

Les résultats de concentrations minimales inhibitrices obtenues pour les souches d'*Escherichia coli* ATCC 35218 ainsi que les souches d'*Escherichia coli* ATCC 25922 sont comparés avec la méthode classique par inondation du milieu dans le tableau 1 ci-dessous. Les valeurs sont données en µg/ml à des temps d'incubation différents. Les valeurs d'inhibition pour la méthode par inondation sont mesurées à partir d'images obtenues par un dispositif de captation par ombroscopie tel que décrit précédemment. Les valeurs d'inhibition pour la méthode selon l'invention sont mesurées à partir d'images obtenues par un dispositif de captation par ombroscopie à 5 heures et visuellement à 7 heures.

**Tableau 1, R=Résistant, valeurs en µg/ml**

| | **Souche** | **ATCC 35218** | | | | | |
|---|---|---|---|---|---|---|---|
| **Gentamicine** | Temps d'incubation | **4H** | **5H** | **6H** | **7H** | **8H** | **24H** |
| | **Inondation** | 0,96 | 1,14 | 1,17 | | 1,17 | 1,06 |
| | **Dépôt en gouttelettes** | | 2 | | 2 | | |
| **Tetracycline** | Temps d'incubation | **4H** | **5H** | **6H** | **7H** | **8H** | **24H** |
| | **Inondation** | 0,44 | 0,86 | 0,96 | | 1,117 | 2,3 |
| | **Dépôt en gouttelettes** | | 1,5 | | 2 | | |
| **Ampicilline/Sulbactam** | Temps d'incubation | | **5H** | **6H** | **7H** | **8H** | **20H** |
| | **Inondation** | | | 10,85 | | 12,29 | 12,3 |
| | **Dépôt en gouttelettes** | | 18 | | 18 | | |
| **Ampicilline** | Temps d'incubation | | **5H** | **6H** | **7H** | **8H** | **18H** |
| | **Inondation** | | | **R** | | **R** | **R** |
| | **Dépôt en gouttelettes** | | R | | R | | |

| | **Souche** | **ATCC 25922** | | | | | |
|---|---|---|---|---|---|---|---|
| **Gentamicine** | Temps d'incubation | **4H** | **5H** | **6H** | **7H** | **8H** | **24H** |
| | **Inondation** | 1,74 | 1,51 | 1,75 | | 1,82 | 1,54 |
| | **Dépôt en gouttelettes** | | 2 | | 2 | | |
| **Tetracycline** | Temps d'incubation | **4H** | **5H** | **6H** | **7H** | **8H** | **24H** |
| | **Inondation** | 0,3 | 0,6 | 0,66 | | 0,86 | 1,77 |
| | **Dépôt en gouttelettes** | | 1,5 | | 2 | | |
| **Ampicilline/Sulbactam** | Temps d'incubation | | **5H** | | **7H** | | |
| | **Inondation** | | | | | | |
| | **Dépôt en gouttelettes** | | 4 | | 6 | | |
| **Ampicilline** | Temps d'incubation | | **5H** | **6H** | **7H** | **8H** | **18H** |
| | **Inondation** | | | 3,43 | | 5,21 | 5,69 |
| | **Dépôt en gouttelettes** | | 8 | | 8 | | |

Le tableau 1 permet de conclure à une bonne corrélation des CMI estimées entre la méthode classique et la méthode selon l'invention. Il est noté que des faibles différences dans les valeurs obtenues peuvent être observées dans le cas où le dépôt des gouttelettes est trop éloigné du grand bord de la bandelette ou réalisé de manière non parallèle au grand bord. Un positionnement optimal à 1mm de la bandelette de l'axe de dépôt de l'échantillon et de façon parallèle au grand bord permet ainsi de limiter des écarts de valeurs de CMI estimées. D'autre part, il est clairement établi que l'éjection de l'échantillon par le pipetteur automatisée PipeJet^{tm} P9 Nanodispenser (bioFluidix) n'empêche pas la pousse des bactéries testées. Cette expérience démontre également qu'il est possible d'estimer une CMI avec un échantillon de volume réduit, le nombre total de microorganismes déposés par milieu de culture étant ici estimé à 100 000 bactéries pour la méthode selon l'invention. Enfin il est observé un très fort contraste optique à 5 heures en ombroscopie et à 7 heures en analyse visuelle, permettant d'accélérer la méthode de détermination de la concentration minimale inhibitrice grâce au dépôt de l'échantillon en gouttelettes. Ce très fort contraste permet également d'envisager l'utilisation de techniques classiques d'analyse d'image afin de déterminer de façon automatisée cette valeur de concentration.

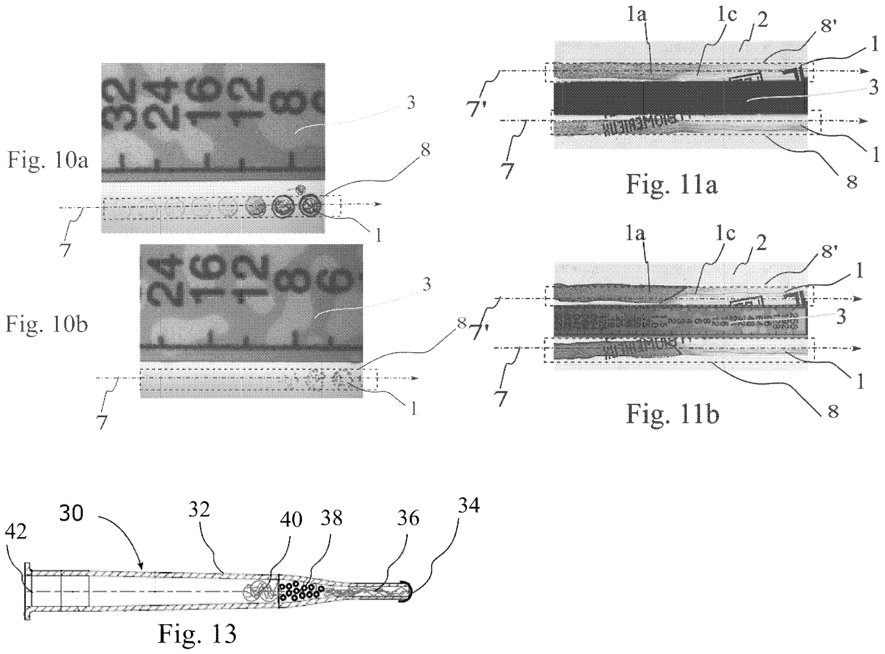

Une seconde expérience de cet exemple est menée afin d'évaluer le comportement de la méthode selon l'invention avec des échantillons de concentrations très faibles. Pour cela, trois suspensions d'*Escherichia coli* ATCC 35218 respectivement à 0,5 Mc Farland, 0,1 Mc Farland et 0,01McFarland sont préparées. Les valeurs de CMI sont évaluées pour chacune de ces souches en présence d'une bandelette de gentamicine, de tétracycline, d'ampicilline/sulbactam ou d'ampicilline. Pour cela, cinquante gouttelettes de 18 nanolitres chacune sont disposées selon un axe parallèle au grand bord de chaque bandelette, de façon similaire à la première expérience et ce pour chacune des valeurs de concentration. La figure 10a illustre une partie de la bandelette et les gouttelettes proches de celle-ci après 5 heures d'incubation de l'échantillon d'*Escherichia coli* ATCC 35218 à 0,5 Mc Farland en présence d'ampicilline/sulbactam. La figure 10b illustre une partie de la bandelette et les gouttelettes proches de celle-ci après 5 heures d'incubation de l'échantillon d'*Escherichia coli* ATCC 35218 à 0,01 Mc Farland en présence d'ampicilline/sulbactam. Les figures 10a et 10b sont obtenues par un dispositif de captation par ombroscopie tel que présenté précédemment. Ces deux figures permettent de déterminer la présence d'une zone d'inhibition et d'estimer une CMI à 10µg/ml et ce même à une concentration très faible. Le tableau 2 suivant présente les résultats de cette seconde expérience en comparaison avec une méthode classique par inondation.

**Tableau 2, R=Résistant, valeurs en µg/ml**

| **Souche** | | **ATCC 35218** | | | | | | |
|---|---|---|---|---|---|---|---|---|
| | | **4H** | **5H** | **6H** | **7H** | **8H** | **18H** | **24H** |
| **Gentamicine** | **inondation** | 0,96 | 1,14 | 1,17 | | 1,17 | | 1,06 |
| **0,5McF** | **gouttelette / replicat 1** | | 2 | | 2 | | | |
| **0,5McF** | **gouttelette/ replicat** 2 | 2 | | 1,5 | | | | |
| **0,1 McF** | **gouttelette** | 2 | | 2 | | | | |
| **0,01 McF** | **gouttelette** | 1,3 | 1,3 | | | | | |
| **Tetracycline** | **inondation** | 0,44 | 0,86 | 0,96 | | 1,117 | | 2,3 |
| **0,5McF** | **gouttelette** | | 1,5 | | 2 | | | |
| **Ampicilline** | **inondation** | | | R | | R | | R |
| **0,5McF** | **gouttelette** | | R | | R | | | |
| **Ampicilline Sulbactam** | **inondation** | | | 10,85 | | 12,29 | | 12,3 |
| **0,5McF** | **gouttelette / replicat 1** | | <16 | | <16 | | | |
| **0,5McF** | **gouttelette/ replicat 2** | 10 | | 10 | | | | |
| **0,1 McF** | **gouttelette** | 10 | | 10 | | | | |
| **0,01 McF** | **gouttelette** | 10 | 10 | | | | | |

Cette seconde expérience permet également de conclure à une bonne corrélation entre les CMI déterminées par la méthode en gouttelettes selon l'invention et la méthode par inondation. Cette expérience démontre également que le procédé selon l'invention permet d'estimer une CMI avec un échantillon de volume réduit et de concentration faible, le nombre total de microorganismes déposés par milieu de culture étant ici estimé à 2000 bactéries, soit 50 gouttes contenant en moyenne 40 bactéries. Enfin il est également observé un très fort contraste optique à 5 heures en ombroscopie et à 7 heures en analyse visuelle, permettant d'accélérer la méthode de détermination de la concentration minimale inhibitrice.

Une troisième méthode de mise en œuvre de l'invention est illustrée par les figures 11a, 11b et 12. Dans cette méthode, l'échantillon est déposé par frottement à la surface du milieu de culture (e.g. gélose dans une boite de Pétri d'un diamètre supérieur ou égal à 9 cm) à l'aide d'un outil de prélèvement tel que décrit dans la demande internationale publiée sous le numéro WO2012/083150. Un tel dispositif intégré permet de réaliser une ou des étapes de filtration ainsi que de transférer un échantillon liquide. Ledit dispositif comprend une partie tubulaire dont l'une des extrémités est recouverte d'un matériau de filtration tel qu'une membrane, ledit matériau de filtration étant disposé à l'extérieur de cette extrémité et la recouvrant totalement. L'autre extrémité de la partie tubulaire est apte à être connectée avec un moyen d'aspiration ou de dispense tel qu'une pompe à vide. L'intérêt de l'utilisation d'un tel outil est notamment de pouvoir estimer la CMI d'un échantillon provenant d'une bouteille d'hémoculture positive, c'est à dire présentant un résultat attestant la présence de microorganismes après un temps d'incubation donné. Pour cela, l'échantillon peut notamment être préparé en prélevant un volume de l'hémoculture positive, en réalisant une étape de lyse des cellules sanguines, par exemple chimique, du volume prélevé, puis en procédant à la filtration par aspiration à la surface d'une membrane du volume lysé. Le concentrât de microorganisme peut alors être déposé directement sur un milieu de culture en apposant la membrane recouvrant l'extrémité de l'outil de prélèvement sur la surface gélosé du milieu de culture puis en la déplaçant à la surface du milieu de culture 2 selon un axe 7 et tel qu'illustré sur la figure 11a. L'opération peut être renouvelée afin de déposer l'échantillon selon un second axe 7'. Une bandelette 3 contenant un agent chimique est déposée parallèlement à l'axe 7 et avantageusement entre les deux axes 7 et 7'. La surface couverte par la ou les zones de dépôt 8, 8' doit alors préférentiellement être adjacente au bord de la bandelette de façon à ne pas être directement en contact avec celle-ci et éviter des effets de diffusion d'agent chimique.

Il va maintenant être décrit un exemple de cette troisième méthode de mise en œuvre de l'invention. Une bandelette 3 Etest® de Gentamicine (bioMérieux) est déposée sur une gélose 2 Mueller Hinton E (bioMérieux, Ref. 413822). Une suspension calibrée à 0,5 Mc Farland d'Escherichia coli ATCC 25922 est préparée.

Afin de réaliser cette opération de façon automatisée, le support de la gélose est disposé sur une platine motorisée se déplaçant en translation selon un axe X, l'axe de la bandelette étant parallèle à cet axe. L'outil de prélèvement est quant à lui supporté par un bras motorisé se déplaçant en translation selon un axe Z sensiblement vertical. En relation avec la figure 13 l'outil de prélèvement 30 comprend un corps 32 de forme tubulaire comprenant une extrémité recouverte d'une membrane 34 PES Supor® d'une porosité de 0,45µm (Pall). L'autre extrémité 42 est débouchante afin de pouvoir connecter un moyen d'aspiration tel qu'une pompe à vide. Dans le corps 32 et entre la membrane 34 et l'extrémité 42 sont disposés : un partie fibreuse 36 tel que du coton, un ensemble de bille de verre 38 dont le diamètre est compris entre 212µm et 300µm, les billes étant maintenues par une partie fibreuse 40 en coton. L'intérêt de ces différentes parties est décrit plus en détail dans la demande de brevet internationale publiée sous le numéro WO2012/083150. Le protocole suivant est effectué afin de préparer l'échantillon :
- 1 mL d'une bouteille d'hémoculture positive est prélevé puis mélangé par trois cycle d'aspiration / dispense à 0,5 mL de tampon de lyse (0.45% w/v Brij-97 + 0.3M CAPS, pH 11.7) à l'aide d'une pipette dans un contenant
- Le mélange est incubé deux minutes à température ambiante afin d'obtenir un échantillon lysé
- Suite à l'incubation, l'extrémité supportant la membrane 34 de l'outil de prélèvement 30 est plongée dans l'échantillon lysé puis filtré par aspiration à -600mbar pendant 2 minutes
- L'outil de prélèvement est ensuite déplacé vers un autre contenant comprenant une première solution de lavage (Brij/Solution Saline (0.45% w/v NaCl + 0.05% Brij 97))
- L'extrémité supportant la membrane 34 de l'outil de prélèvement 30 est plongée dans la première solution de lavage puis aspirée (à -600mbar) pendant 4 minutes
- L'outil de prélèvement est ensuite déplacé vers un autre contenant comprenant une seconde solution de lavage (eau déionisée)
- L'extrémité supportant la membrane 34 de l'outil de prélèvement 30 est plongée dans la seconde solution de lavage puis aspirée (à -600mbar) pendant 3 minutes puis 20 secondes d'aspiration en dehors du contenant afin de limiter l'apparition de bulles dans l'outil de prélèvement 30.
- Les microorganismes contenus dans l'échantillon sont ainsi concentrés à la surface de la membrane 34

Conformément à la figure 11a, l'échantillon 1 ainsi préparé par filtration est alors déposé par déplacement de la membrane 34 en Z de 150µm à partir de la surface de la gélose afin de l'introduire très légèrement dans la gélose. La membrane est ensuite déplacée selon l'axe X sur 65mm à Z constant. Afin de pouvoir mesurer précisément le déplacement en Z de l'outil de prélèvement, des techniques classiques de détection de la surface de la gélose peuvent être employées.

L'échantillon est ainsi déposé selon deux axes 7, 7' de façon adjacente et parallèle aux grands bords de la bandelette 3 Etest® de Gentamicine. Ainsi, les deux zones de dépôt 8, 8' obtenues présentent une répartition uniforme et adjacente aux grands bords de la bandelette.

Chaque zone de dépôt présente une largeur d'environ 2mm. Le milieu de culture ainsi ensemencé est incubé à 35°C. Des images en vue de dessus du milieu sont capturées à l'aide d'un dispositif de captation par ombroscopie à des temps réguliers. Un examen visuel est également effectué. La figure 11a présente une image capturée par ombroscopie après 3h30 d'incubation. La figure 11b présente une image capturée par ombroscopie après 6h30 d'incubation. La figure 12 est une photographie de la même boîte en éclairage naturel après 6h30 d'incubation.

Il est ainsi possible dès 3h30 d'incubation de détecter la présence d'une zone d'inhibition de l'échantillon 1c et d'une zone de croissance des microorganismes présents dans l'échantillon la. Il est également possible de déterminer une concentration minimale inhibitrice de 1,5 µg/ml à partir d'images obtenues par ombroscopie. Un examen visuel à 6h30 confirme également cette valeur telle qu'illustrée sur la figure 12. L'intérêt de cette méthode est donc de pouvoir déterminer une CMI à partir d'un volume d'échantillon très faible et avec un nombre de manipulation restreint et facilement automatisable. Le mouvement de dépôt à la surface de la gélose peut également être effectué par un bras robotisé ou un porte-outil se déplaçant en translation selon trois axes de liberté.

Les procédés et dispositifs décrits dans la présente invention peuvent être mis en œuvre par un ou plusieurs programmes d'ordinateur, qui peuvent être présentés sous des formes diverses, actifs ou inactifs, sur une unique ordinateur ou répartis sur des systèmes d'ordinateur. Par exemple, ils peuvent être mis en œuvre par des logiciels comprenant des instructions aptes à mettre en œuvre les procédés de la présente invention et décrits sous forme de code source, code objet, code exécutable ou tout format permettant la réalisation de certaines étapes des procédés selon l'invention, notamment les étapes consistant à :
- Déterminer la présence ou l'absence de ladite première zone d'inhibition.
- Déterminer le nombre de gouttelettes inhibées dans la zone potentielle d'inhibition et/ou le nombre de gouttelettes non inhibées dans la zone de dépôt de l'échantillon
- Mesurer la distance entre le centre du disque et la première zone d'inhibition afin d'estimer la sensibilité des microorganismes contenus dans l'échantillon à l'agent chimique
- Classer le microorganisme selon une classification à trois critères : Sensible, Intermédiaire ou Résistant, à partir d'un abaque de sensibilité correspondant au microorganisme présent dans l'échantillon et à l'agent chimique, cet abaque étant par exemple disponible sur un support de stockage.
- Localiser une frontière entre la première zone d'inhibition et la zone de croissance des microorganismes
- Déterminer une concentration minimale inhibitrice de l'agent chimique à partir de la localisation de ladite frontière

Tous ces programmes d'ordinateur peuvent être stockés sur un support lisible de stockage pour ordinateur, ce qui inclut les supports de stockages et signaux correspondants, sous une forme compressée ou décompressée.

Le terme ordinateur se réfère à tout dispositif électronique comprenant un processeur, tel qu'une unité centrale de traitement (CPU), un processeur dédié ou un microcontrôleur. Un ordinateur est capable de recevoir des données (une ou des entrées), de réaliser une séquence d'étapes prédéterminées sur ces données, et de produire un résultat sous la forme d'information ou de signaux (une ou des sorties). Selon le contexte, le terme ordinateur peut signifier un processeur en particulier ou plus généralement un processeur associé à un assemblage d'éléments interconnectés contenus dans un boîtier unique.

Le terme support lisible de stockage pour ordinateur ou support de stockage se réfère à tout moyen de contenir, stocker, communiquer, distribuer, ou transporter le programme d'ordinateur pour son utilisation par ou en relation avec un ordinateur ou tout moyen d'exécution dudit programme. Le support lisible de stockage pour ordinateur peut être, de manière non limitative, un système électronique, magnétique, optique, électromagnétique, infrarouge ou à semi-conducteur ainsi qu'un appareil, dispositif ou moyen de propagation dudit programme. Des exemples plus spécifiques et non limitatifs de supports de stockage peuvent être une disquette, un cédérom, de la mémoire vive (RAM), de la mémoire morte (ROM), de la mémoire morte intégrée programmable (EPROM ou stockage FLASH), une fibre optique ou encore toute connexion électrique comprenant un ou plusieurs câbles.

L'invention concerne également un système comprenant un ordinateur ainsi qu'un ou plusieurs programmes d'ordinateur configurés pour mettre en œuvre un ou des procédés selon l'invention. Avantageusement ledit système comprend également des moyens de commande d'un dispositif de captation apte à capturer des images du milieu de culture après incubation, les images capturées étant traitées par ledit programme d'ordinateur. Avantageusement ledit système comprend également des moyens de déplacement du milieu de culture tel que des platines motorisées et les moyens de commandes de ces moyens de déplacement. Avantageusement ledit système comprend également des moyens de dépôt de l'échantillon automatisés tel que des bras robotisés, robot pipetteur, etc.., ainsi que les moyens de commandes de ces moyens de dépôt.

## Revendications

1. Procédé de détection d'une présence ou d'une absence d'au moins une première zone d'inhibition (9) ledit procédé comportant les étapes consistant à :
a. Fournir un milieu de culture gélosé (2) ;
b. Fournir un échantillon (1) contenant ou susceptible de contenir des microorganismes sous forme liquide ;
c. Déposer un volume de l'échantillon (1) sous forme liquide selon une zone de dépôt (8) s'étendant selon un axe (7) à la surface du milieu de culture gélosé ;
d. Effectuer un dépôt d'une quantité déterminée d'un agent chimique (5) à la surface du milieu de culture gélosé (2), ledit dépôt définissant une zone potentielle d'inhibition (6), l'axe de la zone de dépôt de l'échantillon intersectant la zone potentielle d'inhibition ;
e. Incuber ledit milieu de culture gélosé (2);
f. Déterminer la présence ou l'absence de ladite première zone d'inhibition (9);
**caractérisé en ce que** l'étape c) consiste :
- à déposer un volume de l'échantillon sous forme liquide en ligne continue selon une zone de dépôt s'étendant selon un axe à la surface du milieu de culture gélosé ;
- ou à déposer un volume de l'échantillon sous forme liquide en gouttelettes selon une zone de dépôt s'étendant selon un axe à la surface du milieu de culture.

2. Procédé selon la revendication 1 :
- dans lequel le dépôt du volume de l'échantillon est réalisé à l'aide d'une technique de dépôt prédéfinie apte, pour une biomasse de microorganismes donnée dans un volume d'échantillon sous forme liquide, à déposer ledit volume sur une surface maximale du milieu de culture gélosé de manière à obtenir une densité surfacique de microorganismes sensiblement homogène et supérieure à un seuil prédéfini ;
- dans lequel l'échantillon est obtenu au moyen d'une pré-culture d'un échantillon brut, la pré-culture comprenant une phase d'isolement d'une souche de microorganisme suivie d'une phase d'incubation de ladite souche de manière à augmenter la biomasse de microorganismes, ladite biomasse dépendant de la durée d'incubation ; et
- dans lequel la durée d'incubation de l'échantillon brut est choisie inférieure à 10 heures, la surface du dépôt du volume d'échantillon à l'aide de la technique de dépôt prédéfinie étant choisie pour obtenir ladite densité.

3. Procédé selon la revendication 2, dans lequel la durée d'incubation nécessaire pour obtenir une biomasse suffisante pour un dépôt sur la totalité de la surface du milieu de culture gélosé avec ladite densité est supérieure à 20 heures.

4. Procédé de détection selon la revendication précédente, l'échantillon contenant une culture de microorganismes de type connu, l'aire de la zone potentielle d'inhibition étant définie par ledit type de microorganismes

5. Procédé selon la revendication 1, **caractérisé en ce que** la concentration de l'échantillon est comprise entre 0,0005 Me Farland et 0,5 Mc Farland

6. Procédé de détection selon la revendication 1, **caractérisé en ce que** les gouttelettes déposées sont espacées d'un pas prédéterminé P, préférentiellement prédéterminé par l'aire de la zone potentielle d'inhibition et/ou le diamètre des gouttelettes déposées

7. Procédé selon la revendication précédente, **caractérisé en ce que** les gouttelettes déposées sont espacées d'un pas millimétrique, préférentiellement d'un millimètre

8. Procédé de détection selon l'une des revendications 1. 6 ou 7, **caractérisé en ce que** le volume de chaque gouttelette déposée est compris entre 1nL et 10µL

9. Procédé selon l'une des revendications l'une des revendications 1, 6, 7 ou 8, **caractérisé en ce que** la quantité de microorganismes contenue dans chaque goutte déposée est connue et comprise entre 1 microorganisme par goutte et 10⁶ microorganismes par goutte, préférentiellement 10⁴ microorganismes par goutte

10. Procédé selon l'une des revendications l'une des revendications 1, 6-9, **caractérisée en ce qu'**il comprend une étape qui consiste à
- Déterminer le nombre de gouttelettes inhibées (1c) dans la zone potentielle d'inhibition et/ou le nombre de gouttelettes non inhibées (la) dans la zone de dépôt de l'échantillon ;

11. Procédé de détection selon l'une quelconque des revendications précédentes, **caractérisé en ce que** l'étape d) consiste à :
d. Effectuer un dépôt d'un support imprégné (3) d'une quantité déterminée d'un agent chimique à la surface du milieu de culture gélosé, ledit support définissant une zone potentielle d'inhibition, la zone de dépôt de l'échantillon intersectant la zone potentielle d'inhibition ;

12. Procédé de détection selon la revendication précédente, **caractérisé en ce que** le support imprégné est un disque contenant une quantité déterminée d'agent chimique

13. Procédé de détection selon la revendication précédente, **caractérisé en ce qu'**il comprend une étape supplémentaire consistant à :
- Mesurer la distance entre le centre du disque et la première zone d'inhibition afin d'estimer la sensibilité des microorganismes contenus dans l'échantillon à l'agent chimique ;

14. Procédé de détection selon la revendication précédente, **caractérisé en ce qu'**il comprend une étape supplémentaire consistant à :
- Classer le microorganisme selon une classification à trois critères : Sensible, Intermédiaire ou Résistant, à partir d'un abaque de sensibilité correspondant au microorganisme présent dans l'échantillon et à l'agent chimique ;

15. Procédé de détection selon la revendication 12, **caractérisé en ce que** le support imprégné est une bandelette contenant un gradient de concentration d'agent chimique, le dépôt du volume de l'échantillon sous forme liquide étant effectué parallèlement et de façon adjacente au grand bord de ladite bandelette ;

16. Procédé de détection selon la revendication précédente, **caractérisé en ce qu'**il comprend une étape supplémentaire consistant à :
- localiser une frontière entre la première zone d'inhibition et la zone de croissance des microorganismes ;
- déterminer une concentration minimale inhibitrice de l'agent chimique à partir de la localisation de ladite frontière.

## Patentansprüche

1. Verfahren zum Nachweis eines Vorliegens oder einer Abwesenheit mindestens eines ersten Hemmbereichs (9), wobei das Verfahren die folgenden Schritte aufweist:
a. Bereitstellen eines Agar-Nährbodens (2);
b. Bereitstellen einer Probe (1), die Mikroorganismen enthält oder enthalten könnte, in flüssiger Form;
c. Aufbringen eines Volumens der Probe (1) in flüssiger Form gemäß einem Aufbringbereich (8), der sich entlang einer Achse (7) auf der Oberfläche des Agar-Nährbodens erstreckt;
d. Durchführen eines Aufbringens einer bestimmten Menge eines chemischen Mittels (5) auf der Oberfläche des Agar-Nährbodens (2), wobei das Aufbringen einen möglichen Hemmbereich (6) festlegt, wobei die Achse des Aufbringbereichs der Probe den möglichen Hemmbereich schneidet;
e. Inkubieren des Agar-Nährbodens (2);
f. Bestimmen des Vorliegens oder der Abwesenheit des ersten Hemmbereichs (9);
**dadurch gekennzeichnet, dass** der Schritt c) darin besteht:
- ein Volumen der Probe in flüssiger Form als durchgehende Linie gemäß einem Aufbringbereich aufzubringen, der sich entlang einer Achse auf der Oberfläche des Agar-Nährbodens erstreckt;
- oder ein Volumen der Probe in flüssiger Form als Tröpfchen gemäß einem Aufbringbereich aufzubringen, der sich entlang einer Achse auf der Oberfläche des Agar-Nährbodens erstreckt.

2. Verfahren nach Anspruch 1:
- wobei das Aufbringen der Probe mit Hilfe einer vorbestimmten Aufbringtechnik erfolgt, die dazu geeignet ist, bei einer gegebenen Biomasse an Mikroorganismen in einem Probenvolumen in flüssiger Form, dieses Volumen derart auf eine größtmögliche Oberfläche des Agar-Nährbodens aufzubringen, dass eine oberflächenbezogene. Dichte an Mikroorganismen erhalten wird, die im Wesentlichen homogen ist und einen vorbestimmten Schwellenwert übersteigt;
- wobei die Probe mittels eines Vorkulturvorgangs einer Rohprobe erhalten wird, wobei der Vorkulturvorgang eine Phase des Isolierens eines Mikroorganismenstamms umfasst, gefolgt von einer Phase des Inkubierens des Stamms, um die Biomasse an Mikroorganismen zu erhöhen, wobei die Biomasse von der Inkubationsdauer abhängt; und
- wobei die Inkubationsdauer der Rohprobe derart gewählt wird, dass sie geringer als 10 Stunden ist, wobei die Oberfläche der aufgebrachten Bestandteile des Probenvolumens mit Hilfe der vorbestimmten Technik derart gewählt wird, dass diese Dichte erzielt wird.

3. Verfahren nach Anspruch 2, wobei die Inkubationsdauer, welche erforderlich ist, um eine Biomasse zu erhalten, welche für ein Aufbringen auf die Gesamtheit der Oberfläche des Agar-Nährbodens mit einer solchen Dichte ausreicht, mehr als 20 Stunden beträgt.

4. Nachweisverfahren nach dem vorhergehenden Anspruch, wobei die Probe eine Mikroorganismenkultur bekannter Art enthält, wobei die Ausdehnung des möglichen Hemmbereichs anhand der Art von Mikroorganismen festgelegt ist.

5. Verfahren nach Anspruch 1, **dadurch gekennzeichnet, dass** die Konzentration der Probe im Bereich von 0,0005 McFarland bis 0,5 McFarland liegt.

6. Nachweisverfahren nach Anspruch 1, **dadurch gekennzeichnet, dass** die Tröpfchen derart aufgebracht werden, dass sie einen vorbestimmten Wiederholabstand P aufweisen, wobei dieser vorzugsweise anhand der Ausdehnung des möglichen Hemmbereichs und/oder des Durchmessers der aufgebrachten Tröpfchen vorbestimmt wird.

7. Verfahren nach dem vorhergehenden Anspruch, **dadurch gekennzeichnet, dass** die Tröpfchen derart aufgebracht werden, dass sie einen Wiederholabstand im Millimeterbereich aufweisen, welcher vorzugsweise einen Millimeter beträgt.

8. Nachweisverfahren nach einem der Ansprüche 1, 6 oder 7, **dadurch gekennzeichnet, dass** das Volumen jedes aufgebrachten Tröpfchens im Bereich von 1 nL bis 10 µL liegt.

9. Verfahren nach einem der Ansprüche 1, 6, 7 oder 8, **dadurch gekennzeichnet, dass** die Menge an Mikroorganismen, die in einem aufgebrachten Tropfen enthalten ist, bekannt ist und im Bereich von 1 Mikroorganismus pro Tropfen bis 10⁶ Mikroorganismen pro Tropfen liegt, wobei sie vorzugsweise 10⁴ Mikroorganismen pro Tropfen beträgt.

10. Verfahren nach einem der Ansprüche 1, 6 bis 9, **dadurch gekennzeichnet, dass** es einen Schritt umfasst, der darin besteht
- die Anzahl an gehemmten Tröpfchen (1c) im möglichen Hemmbereich und/oder die Anzahl an ungehemmten Tröpfchen (1a) im Hemmbereich im Aufbringbereich der Probe zu bestimmen;

11. Nachweisverfahren nach einem beliebigen der vorhergehenden Ansprüche, **dadurch gekennzeichnet, dass** der Schritt d) darin besteht:
d. ein Aufbringen eines Trägers (3), der mit einer bestimmten Menge eines chemischen Mittels durchtränkt ist, auf der Oberfläche des Agar-Nährbodens durchzuführen, wobei der Träger einen möglichen Hemmbereich festlegt, wobei der Aufbringbereich der Probe den möglichen Hemmbereich schneidet.

12. Nachweisverfahren nach dem vorhergehenden Anspruch, **dadurch gekennzeichnet, dass** es sich bei dem durchtränkten Träger um eine Scheibe handelt, die eine bestimmte Menge an chemischem Mittel enthält.

13. Nachweisverfahren nach dem vorhergehenden Anspruch, **dadurch gekennzeichnet, dass** es einen zusätzlichen Schritt umfasst, der darin besteht:
- die Entfernung zwischen der Mitte der Scheibe und dem ersten Hemmbereich zu messen, um die Empfindlichkeit der Mikroorganismen, die in der Probe enthalten sind, gegenüber dem chemischen Mittel abzuschätzen.

14. Nachweisverfahren nach dem vorhergehenden Anspruch, **dadurch gekennzeichnet, dass** es einen zusätzlichen Schritt umfasst, der darin besteht:
- den Mikroorganismus gemäß einer Abstufung mit drei Kriterien einzustufen: empfindlich, dazwischenliegend oder resistent, ausgehend von einem Empfindlichkeits-Messschieber, der dem Mikroorganismus, welcher in der Probe vorliegt, und dem chemischen Mittel entspricht.

15. Nachweisverfahren nach Anspruch 12, **dadurch gekennzeichnet, dass** es sich bei dem durchtränkten Träger um einen Streifen handelt, der einen Konzentrationsgradienten an chemischem Mittel enthält, wobei das Aufbringen des Volumens der Probe in flüssiger Form parallel zur langen Kante dieses Streifen und an diese angrenzend durchgeführt wird.

16. Nachweisverfahren nach dem vorhergehenden Anspruch, **dadurch gekennzeichnet, dass** es einen zusätzlichen Schritt umfasst, der darin besteht:
- eine Grenzlinie zwischen dem ersten Hemmbereich und dem Wachstumsbereich der Mikroorganismen örtlich zu bestimmen;
- ausgehend von der örtlichen Bestimmung dieser Grenzlinie eine minimale Hemmkonzentration des chemischen Mittels zu bestimmen.

## Claims

1. A method for detecting a presence or an absence of at least one inhibition (9), the method comprising the steps consisting in:
a. providing an agar culture medium (2);
b. providing a sample (1) containing or liable to contain microorganisms in liquid form;
c. depositing a volume of the sample (1) in liquid form along a deposition zone (8) extending along an axis (7) at the surface of the agar culture medium;
d. depositing a determined amount of a chemical agent (5) at the surface of the agar culture medium, the deposit defining a potential zone of inhibition (6), the axis of the zone of deposition of the sample intersecting the potential zone of inhibition;
e. incubating the agar culture medium (2);
f. determining the presence or the absence of the first zone of inhibition;
**characterized in that** the step c) consists in:
- depositing a volume of the sample in liquid form in a continuous line along a deposition zone extending along an axis at the surface of the agar culture medium;
- or depositing a volume of the sample in liquid form in droplets along a deposition zone extending along an axis at the surface of the culture medium.

2. The method as claimed in claim 1:
- in which the volume of the sample is deposited by means of a predefined deposition technique which is suitable, for a given biomass of microorganisms in a volume of sample in liquid form, for depositing the volume over a maximum surface area of the agar culture medium so as to obtain a substantially homogeneous surface density of microorganisms which is greater than a predefined threshold;
- in which the sample is obtained by means of pre-culture of a crude sample, the pre-culture comprising a phase of isolation of a strain of microorganism followed by a phase of incubation of the strain so as to increase the biomass of microorganisms, the biomass depending on the duration of incubation;
- and in which the duration of incubation of the crude sample is chosen to be less than 10 hours, the surface area of deposition of the volume of sample by means of the predefined deposition technique being chosen to obtain the density.

3. The method as claimed in claim 2, in which the duration of incubation necessary to obtain a sufficient biomass for deposition over the whole of the surface of the agar culture medium at the density is greater than 20 hours.

4. The detection method as claimed in the preceding claim, the sample containing a culture of microorganisms of known type, the area of the potential zone of inhibition being defined by the type of microorganisms.

5. The method as claimed in claim 1, **characterized in that** the concentration of the sample is between 0.0005 McFarland and 0.5 McFarland.

6. The detection method as claimed in claim 1, **characterized in that** the deposited droplets are spaced apart by a predetermined interval P, preferably predetermined by the area of the potential zone of inhibition and/or the diameter of the deposited droplets.

7. The method as claimed in preceding claim, **characterized in that** the deposited droplets are spaced apart by an interval in the millimeter range, preferably by one milimeter.

8. The detection method as claimed in claim 1, 6 or 7, **characterized in that** the volume of each deposited droplet is between 1nL and 10µL.

9. The method as claimed in claim 1, 6, 7 or 8, **characterized in that** the amount of microorganisms contained in each deposited drop is known and is between 1 microorganism per drop and 10⁶ microorganisms per drop, preferably 10⁴ microorganisms per drop.

10. The method as claimed in any one of claims 1, 6-9, **characterized in that** it comprises a step which consists in determining the number of inhibited droplets (1c) in the potential zone of inhibition and/or the number of non-inhibited droplets (1b) in the zone of deposition of the sample.

11. The detection method as claimed in anyone of the preceding claims, **characterized in that** the step d) consists in:
d. depositing a support impregnated with a determined amount of a chemical agent at the surface of the agar culture medium, the support defining a potential zone of inhibition, the zone of deposition of the sample intersecting the potential zone of inhibition.

12. The detection method as claimed in the preceding claim, **characterized in that** the impregnated support is a disk containing a determined amount of chemical agent.

13. The detection method as claimed in the preceding claim, **characterized in that** it comprises an additional step consisting in:
- measuring the distance between the center of the disk and the first zone of inhibition so as to estimate the sensitivity of the microorganisms contained in the sample to the chemical agent.

14. The detection method as claimed in the preceding claim, **characterized in that** it comprises an additional step consisting in:
- classifying the microorganism according to a classification containing three criteria: Sensitive, Intermediate or Resistant, from a sensitivity chart corresponding to the microorganism present in the sample and to the chemical agent.

15. The detection method as claimed in claim 12, **characterized in that** the impregnated support is a strip containing a concentration gradient of chemical agent, the volume of the sample in liquid form being deposited parallel and adjacent to the long edge of the strip.

16. The detection method as claimed in the preceding claim, **characterized in that** it comprises an additional step consisting in:
- locating a boundary between the first zone of inhibition and the zone of growth of the microorganisms;
- determining a minimum inhibitory concentration of the chemical agent from the location of the boundary.
